# EUROPEAN PATENT APPLICATION

(11) **EP 4 492 058 A1**
(43) Date of publication of application: **15.01.2025**
(21) Application number: 23184909.2
(22) Date of filing: 12.07.2023
(51) Int. Cl.: G01N 33/50, G01N 33/574

(54) **SIZE DISTRIBUTIONS AND FRACTOGRAMS OF CHOLESTEROL-RICH LIPOPROTEIN NANOPARTICLES FOR CANCER DIAGNOSIS**

(71) Applicant: ECOLE POLYTECHNIQUE FEDERALE DE LAUSANNE (EPFL), 1015 Lausanne (CH)
(72) Inventor: Wei, Lixia, 1015 Lausanne (CH); Ong, Quy, 1015 Lausanne (CH); Stellacci, Francesco, 1015 Lausanne (CH)
(74) Representative: Vossius & Partner Patentanwälte Rechtsanwälte mbB

(57) **Abstract**

The present invention relates to a method for the diagnosis of cancer in a sample of cholesterol-rich lipoprotein nanoparticles obtained from a test subject comprising (α) determining the size distribution or the fractogram in chromatography of the cholesterol-rich lipoprotein nanoparticles in the sample of cholesterol-rich lipoprotein nanoparticles obtained from the test subject, and (β) comparing the size distribution or the fractogram of (α) to the corresponding size distribution or fractogram in one or more samples of cholesterol-rich lipoprotein nanoparticles obtained from one or more healthy subjects or a predetermined standard size distribution or fractogram obtained from a corresponding size distribution or fractogram in one or more samples of cholesterol-rich lipoprotein nanoparticles obtained from one or more healthy subjects, wherein a different size distribution or fractogram in the sample of the test subject as compared to the size distribution or the fractogram in the one or more samples of cholesterol-rich lipoprotein nanoparticles obtained from one or more healthy subjects or the predetermined standard size distribution or fractogram indicates that the test subject has cancer..

## Description

The present invention relates to a method for the diagnosis of cancer in a sample of cholesterol-rich lipoprotein nanoparticles obtained from a test subject comprising (a) determining the size distribution or the fractogram in chromatography of the cholesterol-rich lipoprotein nanoparticles in the sample of cholesterol-rich lipoprotein nanoparticles obtained from the test subject, and (β) comparing the size distribution or the fractogram of (a) to the corresponding size distribution or fractogram in one or more samples of cholesterol-rich lipoprotein nanoparticles obtained from one or more healthy subjects or a predetermined standard size distribution or fractogram obtained from a corresponding size distribution or fractogram in one or more samples of cholesterol-rich lipoprotein nanoparticles obtained from one or more healthy subjects, wherein a different size distribution or fractogram in the sample of the test subject as compared to the size distribution or the fractogram in the one or more samples of cholesterol-rich lipoprotein nanoparticles obtained from one or more healthy subjects or the predetermined standard size distribution or fractogram indicates that the test subject has cancer.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

### INTRODUCTION

Cancer diagnosis is crucial for personal health control and response to treatment. Cancer survival is mainly due to a combination of diagnosis and access to prompt and effective care. It is commonly accepted that early cancer diagnosis is essential for efficient control and response to treatment.

Current methods for cancer diagnosis are based mainly on the detection of cancer biomarkers that in include circulating tumor DNA, mRNA, miRNA, proteins like tumor cell receptors, enzymes, and metabolites. These are of elusively low quantity and shadowed by overly abundant molecules (proteins) in patient samples. Protein substances produced by cancer cells or by the organism in response to cancer have been used for cancer diagnosis. As most protein biomarkers are present in blood which is the biggest body fluid, their detection poses grand challenges.

Unfortunately, currently, for most types of cancers, diagnosis requires some form of imaging, while efficient early diagnosis approaches would rather require the analysis of one or more body fluids directly. It is for this reason that cancer detection from the blood has long been considered the holy grail of cancer diagnosis, unfortunately with very limited success. For instance, for prostate cancer early diagnosis is a known success thanks to the accepted biomarker prostate-specific antigen (PSA). Basically, for all other cancer types, currently no such biomarkers are available. Recently, two approaches have found limited success in finding a correlation between circulating proteins in the blood and a few different types of cancer. They are both based on extracting from the blood objects (extracellular vesicles or nanomaterials) that have adsorbed proteins and then analyzing these proteins through a full proteomic analysis. The key to these methods is that what is extracted from the blood interacts preferentially with lower abundance proteins that carry important health-related information. The main limitation is that currently they all require a large amount of blood and the correlation with cancer is not very strong.

Hence, there is an ongoing need for new approaches for cancer detection, in particular new approaches for cancer detection that overcome one or more of the discussed disadvantages of the existing cancer detection methods. This need is addressed by the present invention.

### DETAILED DESCRIPTION OF THE INVENTION

Accordingly the present invention relates to a method for the diagnosis of cancer in a sample of cholesterol-rich lipoprotein nanoparticles obtained from a test subject comprising (a) determining the size distribution or the fractogram in chromatography of the cholesterol-rich lipoprotein nanoparticles in the sample of cholesterol-rich lipoprotein nanoparticles obtained from the test subject, and (β) comparing the size distribution or the fractogram of (a) to the corresponding size distribution or fractogram in one or more samples of cholesterol-rich lipoprotein nanoparticles obtained from one or more healthy subjects or a predetermined standard size distribution or fractogram obtained from a corresponding size distribution or fractogram in one or more samples of cholesterol-rich lipoprotein nanoparticles obtained from one or more healthy subjects, wherein a different size distribution or fractogram in the sample of the test subject as compared to the size distribution or the fractogram in the one or more samples of cholesterol-rich lipoprotein nanoparticles obtained from one or more healthy subjects or the predetermined standard size distribution or fractogram indicates that the test subject has cancer.

The term "diagnosing" as used herein is directed to the identification of a disease in a subject suffering from symptoms of a disease. In accordance with the invention the disease is a cancer. In this connection it is to be understood that the method of the invention - just as essentially all diagnostic methods that are based on disease markers - provides an indication that the test subject has cancer and no absolute proof. Once a subject is diagnosed as having cancer based on the method of the invention the existence of the cancer can be further verified, for example, based on a biopsy (e.g. a needle biopsy), or an imaging test, such as a computed tomography (CT) scan or a magnetic resonance imaging (MRI) scan.

Cancer is an abnormal malignant new growth of tissue that possesses no physiological function and arises from uncontrolled usually rapid cellular proliferation. The cancer is preferably selected from the group consisting of breast cancer, ovarian cancer, endometrial cancer, vaginal cancer, vulva cancer, bladder cancer, salivary gland cancer, pancreatic cancer, thyroid cancer, kidney cancer, lung cancer, cancer concerning the upper gastrointestinal tract, colon cancer, colorectal cancer, prostate cancer, squamous-cell carcinoma of the head and neck, cervical cancer, glioblastomas, malignant ascites, melanomas, lymphomas and leukemias. Among this list of cancers prostate cancer, multiple melanoma and colon cancer are preferred since their diagnosis is illustrated by the appended examples.

The cancer is preferably a solid cancer. A solid cancer is an abnormal mass of tissue that usually does not contain cysts or liquid areas by contrast to a liquid cancer.

The term "cholesterol-rich lipoprotein nanoparticles" (CRLN) as used herein refers to particles having a size of 10-70 nm that were or can be extracted from blood, plasma, or serum and that contain proteins. Cholesterol-rich lipoprotein nanoparticles are generally made of proteins and fats that carry cholesterol through the bloodstream. Hence, cholesterol-rich lipoprotein nanoparticles can be found in and can be isolated from blood samples, in particular from serum and plasma. Cholesterol-rich lipoprotein nanoparticles are biochemical assemblies with the primary function of transporting hydrophobic lipid molecules in blood plasma or other extracellular fluids.

Since cholesterol-rich lipoprotein nanoparticles are found in blood, the samples with the cholesterol-rich lipoprotein nanoparticles are generally derived or obtainable from a blood sample, such as whole blood, serum or plasma.

A size distribution generally indicates the percentage of particles of a certain size (or in a certain size interval). Means and method for determining the size distribution of lipoprotein-cholesterol nanoparticles are well-known in the art. The particle size distribution of a given material is an important analysis parameter in quality control processes and research applications, as many other product properties are directly related to it. Particle size distribution can be represented either in tabular or in graphical form. A descriptive way of representing a particle size distribution is, for example, a histogram, where the width of a bar corresponds to the lower or upper limit of the size class and the height of the bar corresponds to the quantity in that size class.

Examples of suitable size distribution measurement methods will be provided herein below.

A peak in a fractogram generally indicates the percentage of particles of a certain property related to size and composition. Means and method for determining the fractograms of lipoprotein-cholesterol nanoparticles are well-known in the art (see, for example, Qureshi et al., Analytica Chimica Acta 2009, 654(1):85-91 and Quattrini, Drug Deliv Transl Res. 2021; 11(2): 373-395.). The particle's fractograms is an important analysis parameter in quality control processes and research applications, as many other product properties are directly related to it.

As discussed herein above the lipoprotein-cholesterol nanoparticles from healthy and cancer subjects show similar nanoparticle sizes around 17-19 nm. However, it was surprisingly found that an analysis of the size distribution or fractogram in samples of lipoprotein-cholesterol nanoparticles allows to distinguish samples from healthy and cancer subjects, thereby providing a further means - beyond the proteome - of diagnosing cancer based on samples of the of lipoprotein-cholesterol nanoparticles; see Example 4. This has been verified in the appended examples based on analyzing ~100 samples of lipoprotein-cholesterol nanoparticles from cancer patients and 69 from healthy donors. In addition, the size distributions and fractograms were found to be cancer-type specific. Multiple melanoma, colon cancer and prostate cancer display characteristic size distributions or fractograms.

The size distribution or fractogram analysis of the lipoprotein-cholesterol nanoparticles can be carried out rapidly and at low costs. It is therefore preferably carried out before any additional cancer diagnose, such as the protein level / proteome analysis of the lipoprotein-cholesterol nanoparticles that will be described herein below which provides additional accuracy but is more time consuming and costly.

Many statistical parameters can be derived from a size distribution or a fractogram and that can be used in order to determine whether two or more different size distributions or fractograms are statistically different from each other. The different size distribution or fractogram as referred to herein is therefore preferably a statistically different size distribution or fractogram.

Preferred examples of statistical parameters that can be derived from a size distribution are the cumulative distribution and the percentiles of size distribution. Moreover, fractograms can be converted to size distributions (see, for example, Contado et al. (2007), J Chromatogr A. 2007 Jul 20; 1157(1-2): 321-335.). The statistically different size distribution or fractogram is therefore preferably statistically different cumulative distributions or percentiles between two or more different size distributions or different size distributions that were converted from fractograms.

The sample of isolated cholesterol-rich lipoprotein nanoparticles being subjected to step (a) has been obtained from a test subject for whom it is of interest to know whether the subject has cancer or not. Hence, the test subject may be and is preferably as subject that is supposed to have cancer.

The subject is preferably a vertebrate, more preferably a mammal and most preferably human.

The one or more healthy subjects are subjects that do not have cancer (i.e. a non-cancerous subject). Preferably a healthy subject is defined as subject who is in good general health, not having any mental or physical disorder requiring regular or frequent medication (see "The textbook of pharmaceutical medicine" (2013) by Griffin JP, Posner J, Barker GR. (ed.) 7th edition Wiley Publishers).

Regarding the option of using of a predetermined standard in the method of the invention, it is to be understood that when carrying out the method of the invention it is not required to determine the corresponding size distribution or fractogram in one or more samples of cholesterol-rich lipoprotein nanoparticles obtained from one or more healthy subjects as active methods steps. Instead, also a comparison with a predetermined standard can be made that has been obtained from a corresponding size distribution or fractogram in one or more samples of cholesterol-rich lipoprotein nanoparticles obtained from one or more healthy subjects. For instance, in case in step (a) the size distribution or fractogram was determined in the subsequent step (b) a comparison can be made with the size distribution or fractogram of the healthy control, wherein this size distribution or fractogram is in the form of a predetermined standard, such as a table or graphic representation of size distribution or fractogram. The table or graphic representation can be on piece of paper or electronically stored on a computer or storage medium. The above applies *mutatis mutandis* to the corresponding size protein level as described herein below.

In accordance with a preferred embodiment the size distribution of the cholesterol-rich lipoprotein nanoparticles is determined based on the retention time of the cholesterol-rich lipoprotein nanoparticles in a liquid chromatography, preferably in a fast protein liquid chromatography (FPLC); by size exclusion chromatography (SEC); by sodium dodecyl sulfate-polyacrylamide gel electrophoresis (SDS-PAGE); by differential centrifugal sedimentation; by analytical ultracentrifugation; or based on multi-angle dynamic light scattering.

Among this list fast protein liquid chromatography (FPLC) is preferred since it also allows to highly purify cholesterol-rich lipoprotein nanoparticles, as discussed herein above.

Size exclusion chromatography (SEC) is a chromatographic method in which molecules in solution are separated by their size, and in some cases molecular weight. It is usually applied to large molecules or macromolecular complexes such as proteins and industrial polymers.

Sodium dodecyl sulfate-polyacrylamide gel electrophoresis (SDS-PAGE) is a technique widely used in biochemistry, forensic chemistry, genetics, molecular biology and biotechnology to separate biological macromolecules, usually proteins or nucleic acids, based on the difference of their molecular weight.

Differential centrifugal sedimentation is a common procedure used to separate sub-cellular particles (e.g. nanoparticles, colloidal particles, viruses) based on their sedimentation rate. After each centrifugation, the supernatant (non-pelleted solution) is removed from the tube and re-centrifuged at an increased centrifugal force and/or time.

Analytical ultracentrifugation combines an ultracentrifuge with optical monitoring systems. The sample is detected via ultraviolet light absorption and/or interference optical refractive index sensitive system, monitored by light-sensitive diode array or by film in the older machines. The operator can thus observe the change of sample concentration versus the axis of the rotation profile with time as a result of the applied centrifugal field. With modern instrumentation, these observations are electronically digitized and stored for further mathematical analysis.

Multi-angle dynamic light scattering combines scattering angle information and high-resolution particle size distribution from dynamic light scattering. The particle size distribution analysis can be obtained from dynamic light scattering measurement by an integrated method.

In accordance with a further preferred embodiment the method further comprises (γ) comparing the size distribution of (a) to one or more corresponding size distributions from one or more samples of cholesterol-rich lipoprotein nanoparticles, wherein each of the one or more samples has been obtained from one or more cancer subjects having the same type of cancer or one or more predetermined standard size distributions, wherein each predetermined standard size distribution has been obtained from a sample of cholesterol-rich lipoprotein nanoparticles obtained from one or more cancer subjects having the same type of cancer, wherein a substantially same size distribution in the sample of the test subject as compared to the size distribution of a sample of cholesterol-rich lipoprotein nanoparticles that has been obtained from one or more cancer subjects having the same type of cancer or one of the predetermined standard size distributions indicates that the test subject has the same type of cancer.

As discussed above, it is not only possible to distinguish healthy and cancer subjects by comparing the size distribution of the lipoprotein-cholesterol nanoparticles but also to identify the type of cancer because cancer types display a characteristic size distribution that is different from the size distribution of other cancer types. Hence, a cancer type analysis is further conducted in line with the above more preferred embodiment.

The one or more cancer subjects having the same type of cancer are preferably 5 or more, more preferably 10 or more, and most preferably 25 or more such subjects.

In accordance with a preferred embodiment the method further comprises prior to step (a) the step (a)' extracting the cholesterol-rich lipoprotein nanoparticles from a blood sample, preferably serum or plasma sample of the test subject.

As discussed, herein above cholesterol-rich lipoprotein nanoparticles are found in blood, so that a blood sample is needed to obtain a sample of cholesterol-rich lipoprotein nanoparticles. The above preferred further comprises the active step of obtaining a sample of cholesterol-rich lipoprotein nanoparticles from a blood sample.

The blood sample is preferably serum or plasma. Plasma is the fluid and solute component of blood which does not play a role in clotting. It may be defined as blood serum without the clotting factors, or as blood with all cells and clotting factors removed. Serum includes all proteins with blood clotting; all electrolytes, antibodies, antigens, hormones; and any exogenous substances (e.g., drugs or microorganisms). Serum does not contain white blood cells (leukocytes), red blood cells (erythrocytes), platelets, or, as mentioned, anticlotting factors.

In accordance with a more preferred embodiment the method further comprises prior to steps (a) and (a)' the step (a") of obtaining a blood sample from the test subject.

The method of obtaining a blood sample preferably comprises venipuncture or venepuncture. Venipuncture or venepuncture is the process of obtaining intravenous access for the purpose of venous blood sampling.

In accordance with more preferred embodiment the method further comprises extracting the cholesterol-rich lipoprotein nanoparticles from the blood sample, preferably the serum or plasma sample in step (a)' comprises (i) diluting the blood sample, preferably serum or plasma sample at a ratio of between 1:2 to 1:25, preferably 1:5 to 1:15 and most preferably about 1:10 with a physiological buffer, (ii) passing the diluted blood sample, preferably serum or plasma through a filter having a pore size of between 50 nm and 2.5 µm, preferably between 100 nm and 1.0 µm, and most preferably between 200 nm and 500 nm, (iii) adding 1/2 to 1/10 volume, preferably 1/4 to 1/6 volume and most preferably about 1/5 volume of polyethylene glycol (PEG, preferably having an average molecular weight of 10,000 g/mol and/or at concentration of 50 wt%) to the filtrated diluted blood sample, preferably serum or plasma in order to obtain a PEG mixture, (iv) incubating the PEG mixture for at least 30 min, preferably 30 min to 24h, and most preferably 1h to 3h, at a temperature between 0°C and 37°C, preferably 2°C and 25°C and most preferably at about 4°C, (v) subjecting the incubated PEG mixture to centrifugation under 200 g to 4500 g for at least 5 min, preferably 400 g to 1200 g for 10 min to 20 min and most preferably about 750 g for about 15 min; and (vi) discarding the supernatant and resuspending the pellet comprising the cholesterol-rich lipoprotein nanoparticles from the serum or plasma sample in a physiological buffer.

The term "about" as used herein means for each appearance independently with increasing preference ±10%, ±5% and ±1% of the indicated values.

The above steps for obtaining a sample of cholesterol-rich lipoprotein nanoparticles by extracting the cholesterol-rich lipoprotein nanoparticles from a blood sample are illustrated by the appended examples.

According to this extraction method the sample is initially diluted in physiological buffer which reduces the viscosity of the sample. Physiological buffers can be prepared in the laboratory according to well established methods and are used by the body to prevent large changes in the pH of bodily fluid. The four physiological buffers are the bicarbonate, phosphate, hemoglobin, and protein systems.

The diluted sample is then passed to through a filter having a pore size that allows the cholesterol-rich lipoprotein nanoparticles to pass through the filter while larger and unwanted compounds, such as aggregates stay in the filter. Hence, the filtration step improves the purity of the cholesterol-rich lipoprotein nanoparticles in the sample.

PEG is added of to the filtrated diluted blood sample, preferably serum or plasma in order to obtain a PEG mixture and the subsequent incubation step ensures that the cholesterol-rich lipoprotein nanoparticles bind to PEG.

This makes it possible to centrifuge the PEG mixture in the next step under conditions ensuring that the lipoprotein-cholesterol nanoparticles are in the pellet after centrifugation while unwanted impurities are in the supernatant.

Finally, the supernatant is discarded and the pellet comprising the cholesterol-rich lipoprotein nanoparticles is resuspended in a physiological buffer. The resuspended lipoprotein nanoparticles in the physiological buffer constitute a sample of extracted lipoprotein-cholesterol nanoparticles, wherein the lipoprotein-cholesterol nanoparticles are purified and enriched as compared to the initial sample.

The extracted lipoprotein-cholesterol nanoparticles were analyzed and it was found that the nanoparticles for healthy and cancer subjects overall show similar nanoparticle sizes around 17-19 nm as determined by dynamic light scattering and around 20 nm by visual inspection.

Hence, the above extraction steps result in lipoprotein-cholesterol nanoparticles having a size between the size of low-density lipoprotein (LDL) (20-25 nm) and high-density lipoprotein (HDL) (10-20 nm) as defined by the prior art.

It was furthermore found that a sample of lipoprotein-cholesterol nanoparticles prepared according to the above extraction steps is of superior quality when used in the method of the invention.

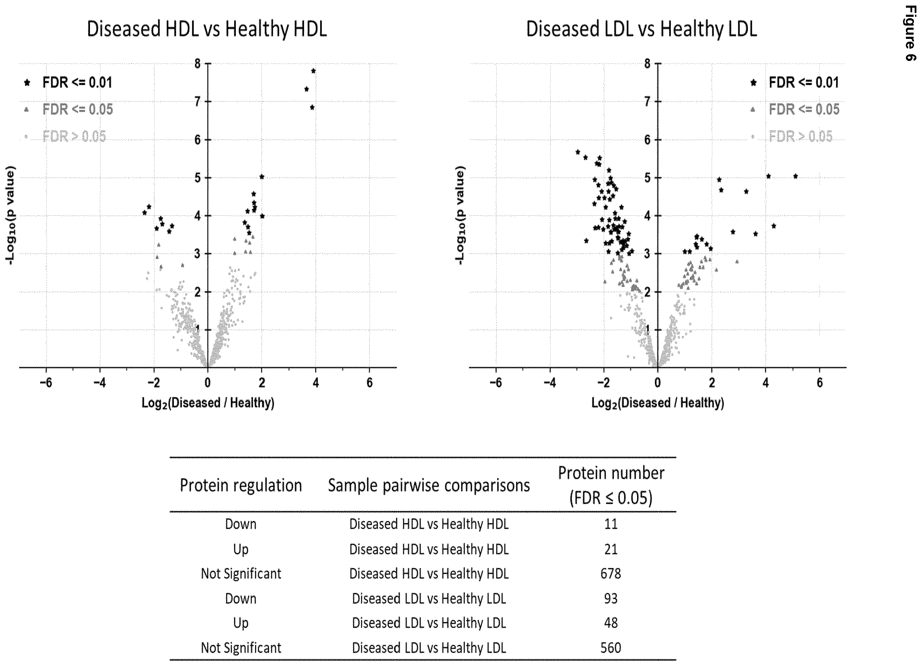

In Example 5 the efficiency of extracting the cholesterol-rich lipoprotein nanoparticles in accordance with the above more preferred embodiment is demonstrated by a comparison with a commercial kit to extract cholesterol-rich lipoprotein nanoparticles. From the result in Figure 6 it can be taken that in the cholesterol-rich lipoprotein nanoparticles as extracted by the commercial kit in total 710 quantified proteins in HDL and 701 proteins quantified in LDL were found. However, among the HDL quantified proteins, only 21 proteins were upregulated, and 11 proteins were downregulated an among the LDL quantified proteins only 48 proteins were upregulated and 93 proteins downregulated. This is much less than the 361 proteins that were found to be either over- or under-expressed in diseased vs. healthy people in the cholesterol-rich lipoprotein nanoparticles that were extracted in accordance with the above more preferred embodiment. Also the number of cholesterol-rich lipoprotein nanoparticles/mL serum is much higher when using the extraction in accordance with the above more preferred embodiment as compared to the commercial kits.

Hence, while also with the commercial kit significantly more differentially expressed proteins as in serum were found, the above extraction steps of the more preferred embodiment result in lipoprotein-cholesterol nanoparticles that are particularly advantageous for the diagnosis of cancer since significantly more differentially expressed proteins are comprised that indicate the presence of cancer.

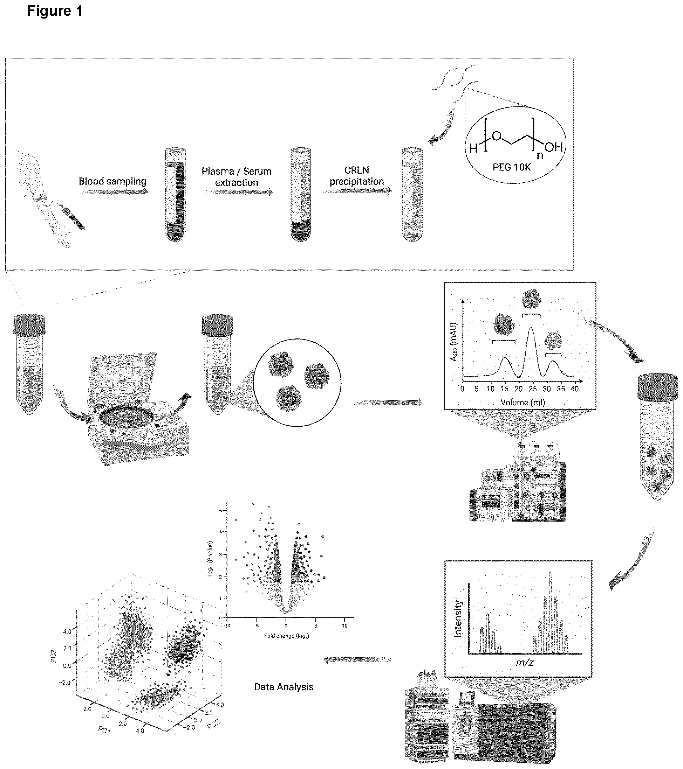

The above extraction steps are also illustrated by Example 1 and Figure 1. This renders it at least plausible that the above extraction steps of the more preferred embodiment also result in lipoprotein-cholesterol nanoparticles that are particularly advantageous for the diagnosis of cancer based on the size distribution or fractogram of these nanoparticles.

In accordance with an even more preferred embodiment the method further comprises after step (i) and before step (ii) (i)' subjecting the diluted blood sample, preferably serum or plasma to centrifugation under 9500 g to 24000 g for at least 15 min, preferably 8500 g to 24000 g for 15 min to 45 min and most preferably about 16000 g for about 30 min and collecting the supernatant as diluted serum or plasma.

This centrifugation step is preferably included because it further removes big aggregates from the diluted blood sample, preferably serum or plasma. The aggregates may block the filter being used in step (ii). The centrifugation conditions ensures that the lipoprotein-cholesterol nanoparticles remain in the supernatant.

In accordance with a further even more preferred embodiment the method further comprises (vii) adding 1/2 to 1/10 volume, preferably 1/4 to 1/6 volume and most preferably about 1/5 volume of PEG (preferably having an average molecular weight of 10,000 g/mol and/or at concentration of 50 wt%) to the resuspended pellet in order to obtain a second PEG mixture, (viii) incubating the second PEG mixture for at least 15 min, preferably 15 min to 24h, and most preferably 20 min to 1h, at a temperature between 0°C and 37°C, preferably 2°C and 25°C and most preferably at about 4°C, (ix) subjecting the incubated PEG mixture to centrifugation under 200 g to 4500 g for at least 5 min, preferably 400 g to 1200 g for 10 min to 20 min and most preferably about 750 g for about 15 min; and (x) discarding the supernatant and resuspending the second pellet comprising the cholesterol-rich lipoprotein nanoparticles from the serum or plasma sample in a physiological buffer.

According to this even more preferred embodiment the extracted lipoprotein-cholesterol nanoparticles, wherein the lipoprotein-cholesterol nanoparticles are purified and enriched as compared to the initial sample, is subjected to a second round of PEG addition, PEG mixture incubation, centrifugation and pellet resuspension in a physiological buffer.

While this second round is not deemed essential, it is expected to further improve the purity and enrichment of the lipoprotein-cholesterol nanoparticles in the sample. This will in turn have a positive effect on the accuracy and/or sensitivity of the cancer diagnosis.

In accordance with a yet further even more preferred embodiment the method further comprising subjected the resuspended pellet or the second resuspended pellet to protein liquid chromatography purification for further purification of the sample of cholesterol-rich lipoprotein nanoparticles.

The further use of protein liquid chromatography purification is preferred since thereby the purity of the lipoprotein-cholesterol nanoparticles in the sample can be further improved. This might again have a positive effect on the accuracy and/or sensitivity of the cancer diagnosis, although the protein liquid chromatography purification is not deemed essential.

Protein liquid chromatography (PLC) is a form of liquid chromatography that is often used to analyze or purify mixtures of proteins. As in other forms of chromatography, separation is possible because the different components of a mixture have different affinities for two materials, a moving fluid (the mobile phase) and a porous solid (the stationary phase). In PLC the mobile phase is an aqueous solution, or buffer. The buffer flow rate is controlled by a positive-displacement pump and is normally kept constant, while the composition of the buffer can be varied by drawing fluids in different proportions from two or more external reservoirs. The stationary phase is a resin composed of beads, usually of cross-linked agarose, packed into a cylindrical glass or plastic column. PLC resins are available in a wide range of bead sizes and surface ligands depending on the application.

In accordance with another even more preferred embodiment the PEG has a molecular weight of 2K to 20K, preferably 5K to 15K and most preferably of about 10K and/or wherein the PEG has a concentration of 25 wt% to 75 wt%, preferably 40 wt% to 60 wt% and most preferably about 50 wt%.

In the examples, 10K PEG having a concentration of 50 wt% is used and PEG having the above molecular wights and/or concentration is also deemed suitable to purify and enrich lipoprotein-cholesterol nanoparticles.

In accordance with an even more preferred embodiment the physiological buffer is a bicarbonate or phosphate buffer, preferably phosphate buffered saline (PBS) and most preferably 1x PBS, pH 7.4.

1x PBS, pH 7.4 is used in the examples and as alternative physiological buffers in particular a bicarbonate buffer or another phosphate buffer can be used as well.

In accordance with an additional more preferred embodiment the serum or plasma sample has a volume of 50µl to 5mL, preferably 100µl to 2mL, and most preferably 400µl to 1mL.

As discussed above, it is a technical advantage of the diagnostic method of the invention that only small amounts of serum or plasma are needed for diagnosing cancer. In the examples, less than 1mL of serum were used and cancer was detected with high accuracy.

In accordance with a preferred embodiment the method further comprises (a) determining the protein level of at least one protein in the sample of isolated cholesterol-rich lipoprotein nanoparticles; and (b) comparing the determined protein level to the corresponding protein level in one or more samples of cholesterol-rich lipoprotein nanoparticles obtained from one or more healthy subjects or a predetermined standard obtained from a corresponding protein level in one or more samples of cholesterol-rich lipoprotein nanoparticles obtained from one or more healthy subjects, wherein an at least 1.5-fold increase or decrease of the determined protein level as determined in (a) as compared to the protein level in one or more samples of cholesterol-rich lipoprotein nanoparticles obtained from one or more healthy subjects or the predetermined standard indicates that the test subject has cancer, and wherein steps (a) and (b) are preferably carried out after step (a) and, if step (a)' is present, after step (a)..

In accordance with a more preferred embodiment the at least one protein is at least 5, preferably at least 10, and most preferably at least 25 proteins.

As discussed herein above, in the lipoprotein-cholesterol nanoparticle samples between 361 differentially expressed proteins that differentiate cancer diseased from healthy subjects were found. It is therefore possible to base the diagnosis of cancer by the method of the invention on the protein levels of at least 5, preferably at least 10, and most preferably at least 25 different proteins. It is also possible and envisioned herein to base the diagnosis of cancer by the method of the invention on the protein levels of at least 5, preferably at least 10, and most preferably at least 25 different upregulated proteins and/or at least 5, preferably at least 10, and most preferably at least 25 different downregulated proteins. Such "cancer fingerprinting" based on a plurality of protein levels of different proteins will increase the accuracy and/or sensitivity of the cancer diagnosis to almost certainty.

The above proteins are preferably selected from the below Tables 1 to 6. In this respect it is of note that Tables 1, 3 and 5 list upregulated proteins while Tables 2, 4 and 6 list downregulated proteins.

Tables 1 and 2 list upregulated and downregulated proteins that were found in subjects having colon cancer, so that these proteins are particularly useful for the diagnosis of colon cancer.

Tables 3 and 4 list upregulated and downregulated proteins that were found in subjects having multiple myeloma, so that these proteins are particularly useful for the diagnosis of multiple myeloma.

Tables 5 and 6 list upregulated and downregulated proteins that were found in subjects having prostate cancer, so that these proteins are particularly useful for the diagnosis of prostate cancer.

**Table 1 - Colon cancer vs. healthy subjects upregulated proteins**

| Protein Name | Gene.names | Fold Change | LIMMA_p_value | FDR |
|---|---|---|---|---|
| ITIH4 protein | ITIH4 | 10,02015252 | 9,49E-08 | FDR <= 0.01 |
| Beta-2-glycoprotein 1 | APOH | 7,778864554 | 1,886-07 | FDR <= 0.01 |
| Complement C1s subcomponent | C1S | 5,636178698 | 1,83E-07 | FDR <= 0.01 |
| Haptoglobin | HP | 5,166797392 | 0,000124517 | FDR <= 0.01 |
| Serotransferrin | TF | 5,153665813 | 2,87E-07 | FDR <= 0.01 |
| Serum amyloid P-component | APCS | 4,868142315 | 7,50E-05 | FDR <= 0.01 |
| Plasma kallikrein | KLKB1 | 4,804835519 | 1,13E-05 | FDR <= 0.01 |
| Isoform 2 of Mannan-binding lectin serine protease 1 | MASP1 | 4,772443274 | 2,10E-06 | FDR <= 0.01 |
| C3/C5 convertase | C2 | 4,535472255 | 2,54E-06 | FDR <= 0.01 |
| Mannan-binding lectin serine protease 1 | MASP1 | 4,104257638 | 1,03E-06 | FDR <= 0.01 |
| Hemopexin | HPX | 3,821703163 | 3,13E-06 | FDR <= 0.01 |
| Complement component C9 | C9 | 3,706789993 | 3,75E-06 | FDR <= 0.01 |
| Platelet basic protein | PPBP | 3,671586199 | 1,89E-06 | FDR <= 0.01 |
| Alpha-1-acid glycoprotein 1 | ORM1 | 3,527964969 | 0,000399859 | FDR <= 0.01 |
| Apolipoprotein A-II | APOA2. | 3,507050285 | 3,99E-05 | FDR <= 0.01 |
| Immunoglobulin heavy constant gamma 2 | IGHG2 | 3,499197683 | 0,00036764 | FDR <= 0.01 |
| Apolipoprotein C-III | APOC3 | 3,449609332 | 0,004032062 | FDR <= 0.05 |
| Coagulation factor IX | F9 | 3,441767007 | 2.74E-05 | FDR <= 0.01 |
| Complement factor I | CFI | 3,337571644 | 1,68E-06 | FDR <= 0.01 |
| Kininogen-1 | KNG1 | 3,32468328 | 1,46E-05 | FDR <= 0.01 |
| Prothrombin | F2 | 3,313126812 | 1,52E-05 | FDR <= 0.01 |
| Low affinity immunoglobulin gamma Fc region receptor III-A | FCGR3A | 3,177939401 | 0,003012226 | FDR <= 0.05 |
| Complement component C6 | C6 | 2,927094394 | 8,36E-06 | FDR <= 0.01 |
| Immunoglobulin heavy constant gamma 1 | IGHG1 | 2,925673325 | 0,0021809 | FDR <= 0.05 |
| Apolipoprotein A-I | APOA1 | 2,836078799 | 0,000133959 | FDR <= 0.01 |
| Tetranectin | CLEC3B | 2,794092688 | 7,90E-05 | FDR <= 0.01 |
| Complement component C8 alpha chain | C8A | 2,70418385 | 4,10E-05 | FDR <= 0.01 |
| Afamin | AFM | 2,653505961 | 3,83E-05 | FDR <= 0.01 |
| Antithrombin-III | SERPINC1 | 2,636244535 | 6,87E-05 | FDR <= 0.01 |
| Plasma protease C1 inhibitor | SERPING1 | 2,601023933 | 6,18E-05 | FDR <= 0.01 |

**Table 2 - Colon cancer vs. healthy subjects downregulated proteins**

| Protein Name | Gene.names | Fold Change | LIMMA_p_value | FDR |
|---|---|---|---|---|
| ARP2 actin-related protein 2 homolog (Yeast), isoform CRA_d | ACTR2 | 7,57755426 | 4,87E-07 | FDR <= 0.01 |
| Alpha-actinin-1 | ACTN1 | 7,105370376, | 7,11E-06 | FDR <= 0.01 |
| Caveolae-associated protein 2 | CAVIN2 | 6,695953024 | 0,000116488 | FDR <= 0.01 |
| Talin-1 | TLN1 | 6,567879972 | 5,27E-07 | FDR <= 0.01 |
| Intelectin-1 | ITLN1 | 6,201569848 | 8,99E-08 | FDR <= 0.01 |
| Tropomyosin alpha-4 chain | TPM4 | 6,052647489 | 5,90E-05 | FDR <= 0.01 |
| Adenylyl cyclase-associated protein 1 | CAP1 | 5,892957943 | 0,000332804 | FDR <= 0.01 |
| DNA-directed RNA polymerase II subunit GRINL1A | GCOM1 | 4,319170811 | 1,19E-05 | FDR <= 0.01 |
| Vasodilator-stimulated phosphoprotein | VASP | 4,021271529 | 9,30E-05 | FDR <= 0.01 |
| Ras-related protein Rap-1b | RAP1B | 3,70475289 | 5,51E-06 | FDR <= 0.01 |
| 59 kDa serine/threonine-protein kinase | ILK | 3,610484563 | 0,000148088 | FDR <= 0.01 |
| Tropomyosin beta chain | TPM2 | 3,487321197 | 0,001943925 | FDR <= 0.05 |
| Bridging integrator 2 | BIN2 | 3,425076696 | 0,000113593 | FDR <= 0.01 |
| Calnexin | CANX | 3,261938098 | 0,014005233 | FDR <= 0.05 |
| Neurogenic locus notch homolog protein 2 | NOTCH2 | 3,04109821 | 4,09E-05 | FDR <= 0.01 |
| Thrombospondin-4 | THBS4 | 3,000943968 | 0,000542947 | FDR <= 0.01 |
| Immunoglobulin heavy variable 1-69D | IGHV1-69D | 2,934202295 | 0,001783815 | FDR <= 0.01 |
| Proprotein convertase 9 | PCSK9 | 2,860715368 | 0,000172218 | FDR <= 0.01 |
| Tropomyosin alpha-3 chain | TPM3 | 2,780428329 | 0,000124268 | FDR <= 0.01 |
| Complement C4-A | C4A | 2,777551816 | 0,00031747 | FDR <= 0.01 |
| Keratin, type I cytoskeletal 19 | KRT19 | 2,760922.926 | 0,000207139 | FDR <= 0.01 |
| Phosphatidylinositol-glycan-specific phospholipase D | GPLD1 | 2,740779553 | 1,11E-05 | FDR <= 0.01 |
| Apolipoprotein B-100 | APOB | 2,70532201 | 0,000830428 | FDR <= 0.01 |
| Protein-glutamine gamma-glutamyltransferase 2 | TGM2 | 2,679131867 | 0,010995846 | FDR <= 0.05 |
| Dermcidin | DCD | 2,640695532 | 0,007067005 | FDR <= 0.05 |
| Angiopoietin-related protein 6 | ANGPTL6 | 2,63605829 | 0,0000208 | FDR <= 0.01 |
| Transitional endoplasmic reticulum ATPase | VCP | 2,56355863 | 0,005235738 | FDR <= 0.05 |
| Extracellular matrix protein 1 | ECM1 | 2,540759731 | 0,0000354 | FDR <= 0.01 |
| Thioredoxin domain-containing protein 5 | TXNDC5 | 2,51128592 | 0,0000881 | FDR <= 0.01 |
| Cartilage oligomeric matrix protein | COMP | 2,504109099 | 0,000559202 | FDR <= 0.01 |

**Table 3 - Multiple myeloma vs. healthy subjects downregulated proteins**

| Protein Name | Gene.nanies | Fold Change | LIMMA_p_value | FDR |
|---|---|---|---|---|
| Intelectin-1 | ITLN1 | 6,319280524 | 5,52E-05 | FDR <= 0.01 |
| Lipocalin-1 | LCN1 | 5,646738939 | 0,001749919 | FDR <= 0.05 |
| Serglycin | SRGN | 4,827987752 | 3,26E-05 | FDR <= 0.01 |
| Actin-related protein 2/3 complex subunit 16 | ARPC1B | 3,600920146 | 0,002117868 | FDR <= 0.05 |
| Protein-glutamine gamma-glutamyltransferase 2 | TGM2 | 3,382353627 | 0,000214449 | FDR <= 0.01 |
| Coronin | COR01C | 3,325926395 | 0,000247646 | FDR <= 0.01 |
| Extracellular matrix protein 1 | ECM1 | 3,307555995 | 6,31E-05 | FDR <=0.01 |
| Immunoglobulin heavy variable 3/OR16-10 | IGHV3OR16-10 | 3,252472614 | 2.07E-05 | FDR <= 0.01 |
| Clathrin heavy chain 2 | CLTCL1 | 3,021443517 | 0,00242298, | FDR <= 0.05 |
| Drebrin-like protein | DBNL | 2,917726258 | 0,003224028 | FDR <= 0.05 |
| Proprotein convertase 9 | PCSK9 | 2,902743843 | 0,000483597 | FDR <= 0.01 |
| 155 Mg(2+)-ATPase p97 subunit | VCP | 2,721234279 | 0,003454145 | FOR <= 0.05 |
| Adenylyl cyclase-associated protein 1 | CAP1 | 2,63799551 | 0,000168606 | FDR <= 0.01 |
| Fibronectin | FN1 | 2,5978.16514 | 0,00415857 | FDR <= 0.05 |
| Alpha-actinin-1 | ACTN1 | 2,567444561 | 0,008906994 | FDR <= 0.05 |
| Cartilage acidic protein 1 | CRTAC1 | 2,487317188 | 0,001286405 | FDR <= 0.01 |
| Complement factor H | CFH | 2,452625154 | 0,003472779 | FDR <= 0.05 |
| Cartilage oligomeric matrix protein | COMP | 2,22334858 | 0,000512199 | FDR <=0.01 |
| Bleomycin hydrolase | BLMH | 2,167924834 | 0,007718983 | FDR <= 0.05 |
| Integrin alpha-IIb | ITGA2B | 2,154382689 | 0,002735308 | FDR <= 0.05 |
| Isoform 2 of Tropomyosin alpha-3 chain | TPM3 | 2,11002679 | 0,004260472 | FDR <= 0.05 |
| Protein disulfide-isomerase | P4HB | 2,106705218 | 0,007583337 | FDR <= 0.05 |
| A disintegrin and metalloproteinase with thrombospondin motifs 13 | ADAMTS13 | 2,105131213 | 0,002418724 | FDR <= 0.05 |
| 59 kDa serine/threonine-protein kinase | ILK | 2,104672055 | 0,004914511 | FDR <= 0.05 |
| Actinin, alpha 4, isoform CRA_a | ACTN4 | 2,099365303 | 0,001662454 | FDR ε= 0.05 |
| Immunoglobulin lambda-like polypeptide 1 | IGLL1 | 2,042262398 | 0,000948007 | FDR <= 0.01 |
| Beta-Ala-His dipeptidase | CNDP1 | 2,004636934 | 0,00305404 | FDR <= 0.05 |
| Fibrillin-1 | FBN1 | 1,966139894 | 0,001988677 | FDR <= 0.05 |
| Amyloid-beta A4 protein | APP | 1,963401748 | 0,006817536 | FDR <= 0.05 |
| Sex hormone-binding globulin | SHBG | 1,939349851 | 0,005109027 | FDR <= 0.05 |

**Table 4 - Multiple myeloma vs. healthy subjects upregulated proteins**

| Protein Name | Gene.names | Fold Change | LIMMA_p_value | FDR |
|---|---|---|---|---|
| Beta-2-glycoprotein 1 | APOH | 13,05296821 | 7,24E-09 | FDR <= 0.01 |
| Serotransferrin | TF | 9,826638289 | 5,32E-O9 | FDR <= 0.01 |
| Apolipoprotein C-III | APOC3 | 7,431272537 | 5,21E-05 | FDR <= 0.01 |
| Apolipoprotein C-I | APOC1 | 7,234146614 | 2,42E-05 | FDR <= 0.01 |
| Vitamin K-dependent protein C | PROC | 7,168968667 | 5,48E-09 | FDR <= 0.01 |
| Apolipoprotein A-II | APOA2 | 7,110663673 | 7,29E-07 | FDR <= 0.01 |
| ITIH4 protein | ITIH4 | 6,58182335 | 4,35E-05 | FDR <= 0.01 |
| Apolipoprotein A-I | APOA1 | 5,938412479 | 1,78E-06 | FDR <= 0.01 |
| C3/C5 convertase | C2 | 5,902019325 | 1.08E-06, | FDR <= 0.01 |
| Platelet factor 4 | PF4 | 5,338,086852 | 0,002385256 | FDR <= 0.05 |
| Afamin | AFM | 5,223756811 | 7,80E-07 | FDR <= 0.01 |
| Complement C1s subcomponent | C1S | 5,202402657 | 2,83E-06 | FDR <= 0.01 |
| Leucine-rich alpha-2-glycoprotein | LRG1 | 4,773246329 | 2,37E-07 | FDR <= 0.01 |
| Hyaluronan-binding protein 2 | HABP2 | 4,643536312 | 0,000450265 | FDR <= 0.01 |
| Hemopexin | HPX | 4,618623443 | 3,46E-07 | FDR <= 0.01 |
| Mannan-binding lectin serine protease 1 | MASP1 | 4,456759187 | 4.16E-05 | FDR <= 0.01 |
| Complement component C9 | C9 | 4,409378229 | 0,000114371 | FDR <= 0.01 |
| Cathelicidin antimicrobial peptide | CAMP | 4,406132435 | 8,26E-05 | FDR <= 0.01 |
| Alpha-1-acid glycoprotein 1 | ORM1 | 4,202498935 | 0,000115617 | FDR <= 0.01 |
| Plasma protease C1 inhibitor | SERPING1 | 4,106421414 | 6,69E-05 | FDR <= 0.01 |
| Apolipoprotein M | APOM | 4,099203334 | 5,97E-05 | FDR <= 0.01 |
| Antithrombin-III | SERPINC1 | 4,071767926 | 7,41E-06 | FDR <= 0.01 |
| Isoform 2 of Mannan-binding lectin serine protease 1 | MASP1 | 3,983540621 | 3,71E-06 | FDR <= 0.01 |
| Heparin cofactor 2 | SERPIND1 | 3,927146832 | 4,32E-05 | FDR <= 0.01 |
| Hemoglobin subunit alpha | HBA1 | 3,845007242 | 0,010859931 | FDR <= 0.05 |
| Plasma kallikrein | KLKB1 | 3,815134507 | 3,87E-05 | FDR <= 0.01 |
| Kininogen-1 | KNG1 | 3,706323779 | 0,000171528 | FDR <= 0.01 |
| Haptoglobin | HP | 3,480505836 | 2,73E-05 | FDR <= 0.01 |
| Alpha-2-HS-glycoprotein | AHSG | 3,447527651 | 3,95E-05 | FDR <= 0.01 |
| Gc-globulin | GC | 3,34012838 | 0,000247627 | FOR <= 0.01 |

**Table 5 - Prostate cancer vs. healthy subjects downregulated proteins**

| Protein Name | Gene.names | Fold Change | LIMMA_p_value | FDR |
|---|---|---|---|---|
| Intelectin-1 | ITLN1 | 6,896141523 | 2,38E-08 | FDR <= 0.01 |
| Serum amyloid A protein | SAA1 | 5,985707985 | 5,72E-07 | FDR <= 0.01 |
| Isoform LMW of Kininogen-1 | KNG1 | 3,646195623 | 3,56E-06 | FDR <= 0.01 |
| Carboxypeptidase N subunit 2 | CPN2 | 3,623975728 | 3,076-05 | FDR <= 0.01 |
| Clusterin | CLU | 2,904336947 | 1.03E-05 | FDR <= 0.01 |
| Involucrin | IVL | 2,836340464 | 0,003413534 | FDR <= 0.05 |
| Alpha-2-macroglobulin | A2M | 2,833434675 | 0,001038415 | FDR <= 0.01 |
| Complement component C8 gamma chain | C8G | 2,763295698 | 2,14E-05 | FDR <= 0.01 |
| Apolipoprotein A-V | APOA5 | 2,753609879 | 0,000275674 | FDR <= 0.01 |
| Angiopoietin-related protein 6 | ANGPTL6 | 2,679336678 | 0,000658342 | FDR <= 0.01 |
| Insulin-like growth factor-binding protein complex acid labile subunit | IGFALS | 2,485903349 | 0,000100356 | FDR <= 0.01 |
| EGF-containing fibulin-like extracellular matrix protein 1 (Fragment) | EFEMP1 | 2,304090819 | 0,000154186 | FDR <= 0.01 |
| Carboxypeptidase N catalytic chain | CPN1 | 2,191048611 | 0,00172043 | FDR <= 0.05 |
| Alpha-1-antitrypsin | SERPINA1 | 2,153593853 | 0,001587566 | FDR <= 0.05 |
| Phosphatidylinositol-glycan-specific phospholipase D | GPLD1 | 2,152743335 | 0,001990638 | FDR <= 0.05 |
| Cartilage acidic protein 1 | CRTAC1 | 2,100497417 | 0,000276708 | FDR <= 0.01 |
| Transthyretin | TTR | 1,804242969 | 0,002664833 | FDR <= 0.05 |
| Plasminogen | PLG | 1,788085728 | 0,000560379 | FDR <= 0.01 |
| Serum paraoxonase/lactonase 3 | PON3 | 1,637393858 | 0,001926607 | FDR <= 0.05 |

**Table 6 - Prostate vs. healthy subjects upregulated proteins**

| Protein Name | Gene.names | Fold Change | LIMMA_p_value | FDR |
|---|---|---|---|---|
| ITIH4 protein | ITIH4 | 10,10421776 | 1,49E-09 | FDR <= 0.01 |
| Complement C1s subcomponent | C1S | 8,60957482 | 8,02E-08 | FOR <= 0.01 |
| Mannan-binding lectin serine protease 1 | MA5P1 | 6,134418957 | 1,10E-08 | FDR <= 0.01 |
| Neuropilin-2 | NRP2 | 6,072467888 | 3,49E-O7 | FDR <= 0.01 |
| Apolipoprotein F | APOF | 5,313449114 | 0,000344392 | FDR <= 0.01 |
| Mannose-binding protein C | MBL2 | 5,114586867 | 0,000527465 | FDR <= 0.01 |
| Antithrombin-III | SERPINC1 | 5,067335843 | 2,34E-06 | FDR <= 0.01 |
| Isoform 2 of Mannan-binding lectin serine, protease 1 | MASP1 | 4,784489848 | 2,43E-07 | FDR <= 0.01 |
| Isoform C of Fibulin-1 | F8LN1 | 4,749222383 | 5,11E-06 | FDR <= 0.01 |
| Plasma protease C1 inhibitor | SERPING1 | 4,673590897 | 8,17E-08 | FDR <= 0.01 |
| Asporin | ASPN | 3,954613643 | 0,000607145 | FDR <= 0.01 |
| Follistatin-related protein 1 | FSTL1 | 3,920844535 | 4,92E-06 | FDR <= 0.01 |
| Prothrombin | F2 | 3,666809933 | 4,75E-06 | FDR <= 0.01 |
| Plasma kallikrein | KLKB1 | 3,598894413 | 1,30E-06 | FDR <= 0.01 |
| Vitamin K-dependent protein 5 | PROS1 | 3,435303328 | 0,000167813 | FDR <= 0.01 |
| Mannan-binding lectin serine protease 2 | MA5P2 | 3,056312006 | 4,20E-05 | FDR <= 0.01 |
| Gelsolin | GSN | 2,992191957 | 4,75E-06 | FDR <= 0.01 |
| Coagulation factor IX | F9 | 2,943287885 | 2,66E-05 | FDR <= 0.01 |
| Apolipoprotein C-IV | APOC4 | 2,905017134 | 0,00569922 | FDR <= 0.05 |
| Isoform F of Proteoglycan 4 | PRG4 | 2,832729536 | 0,0002307 | FDR <= 0.01 |
| Vimentin | VIM | 2,726465907 | 2,04E-05 | FDR <= 0.01 |
| Coagulation factor X | F10 | 2,722317171 | 5,74E-06 | FDR <= 0.01 |
| Neuropilin | NRP1 | 2,713153676 | 0,00010093 | FDR <= 0.01 |
| Serum amyloid P-component | APC5 | 2,6808565 | 0,000517091 | FDR <= 0.01 |
| Fibulin-1 | FBLN1 | 2,553600025 | 6,53E-05 | FDR <= 0.01 |
| Ficolin-3 | FCN3 | 2,5342.20476 | 7,33E-05 | FDR <= 0.01 |
| Immunoglobulin heavy constant alpha 2 | IGHA2 | 2,493583496 | 2,15E-05 | FDR <= 0.01 |
| Myosin-9 | MYH9 | 2,469886204 | 0,000151878 | FDR <= 0.01 |
| Platelet basic protein | PPBP | 2,241126492 | 0,001138187 | FDR <= 0.01 |
| Filamin-A | FLNA | 2,20697868.5 | 0.000211428 | FDR <= 0.01 |

In accordance with a preferred embodiment the one or more healthy subjects are 5 or more, preferably 10 or more, and most preferably 25 or more healthy subjects.

While the size distribution / fractogram and the proteome of lipoprotein-cholesterol nanoparticles are distinct between cancer and healthy subjects it may be advantageous to base the healthy control on 5 or more, preferably 10 or more, and most preferably 25 or more healthy subjects in order to balance for potential size distribution / fractogram differences and/or proteome differences among samples of lipoprotein-cholesterol nanoparticles from healthy subjects. This is expected to further improve the accuracy and/or sensitivity of the cancer diagnosis.

As regards the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The figures show.
**Fig 1**. Schematic illustration of the cholesterol rich lipoprotein nanoparticle (CRLN) extraction process and proteomic analysis.
**Fig 2****.** Characterization of extracted CRLN. A) SDS-PAGE Gel of five healthy serum vs. five cancer serum (left) and five healthy CRLN-H samples vs. five cancer CRLN-MM samples (right). B) DLS characterization of both healthy CRLN-H and cancer CRLN-MM. C) Representative TEM image of CRLN from healthy donors, scale car: 100 nm.
**Fig 3****.** Principal component analysis (left) and volcano plots (right) of proteomics data for serum and extracted CRLN for healthy (grey round dot) and diseased patients (black star). The healthy are made from the pooled sample, while the diseased come from single individuals.
**Fig 4****.** CRLN-Proteomic Analysis. A) Colon Carcinoma (CC) vs Healthy (H), B) Metastatic Melanoma (MM) vs Healthy (H), C) Prostate Cancer (PC) vs Healthy (H).
**Fig 5****.** A size exclusion fractogram spectrum of CRLN extracted from the serum of healthy donors and three different types of cancer patients reveals that the size distribution of the extracted CRLN is very different for all four types of patients.
**Fig 6****.** Volcano plots of proteomics data for extracted CRLN of the proposed method, in comparison to HDL and LDL from a commercial kit. The sera were obtained from MM diseased patients and pooled healthy donors.

The Examples illustrate the claimed invention.

### Example 1 - CRLN characterization Sample from metastatic melanoma (MM) and Healthy (H)

### Materials

Melanoma Cancer patients' serum are from our collaborator Prof. Daniel Speiser (University of Lausanne, Switzerland). In total 5 samples, each sample contains 1 mL serum (two from female age 39 and 44, three from male age 28, 29, and 59, the cancer stage is late stage).

Protein BSA and pooled serum from healthy donors were purchased from Sigma Aldrich (Missouri, United States). Chemical polyethylene glycol (PEG) 10000 was purchased from Sigma Aldrich (Missouri, United States). PBS pH 7.4 (1X) was purchased from Life Technology (California, United States). NuPAGE^{™} 4 to 12% Bis-Tris gel was purchased from Thermo Fisher (Massachusetts, United States). Unless otherwise noted, all chemical and biological reagents were used as received. All solvents purchased were reagent grade.

### Methods

### Extraction of cholesterol-rich lipoprotein nanoparticles (CRLN)

1 mL serum sample was first to dilute 10 times into 10 mL with PBS 7.4 1X, and then centrifuge under 12000 rpm for 30 mins to remove big aggregates in the serum. After centrifugation, serum was first filtered with 0.22 µm membrane and then adding 1/5 volume of serum (2 mL) 50% (W/V) PEG 10K, mix well, and leave it at 4 degrees for 1 hour. Centrifuge the serum with PEG mixture under 2000 rpm for 15 mins and discard the supernatant. Resuspend the precipitates with PBS 7.4 1X into 5 mL, adding 1/5 volume of the serum solution (1 mL) 50% (W/V) PEG 10K; the second time, mix well and leave it at 4 degrees for 30 mins. Centrifuge the serum with PEG mixture under 2000 rpm for 15 mins, and discard the supernatant. Resuspend the precipitates with PBS 7.4 1X into 1 mL for further Fast Protein Liquid Chromatography (FPLC) SEC column purification.

### Fast protein liquid chromatography purification

An automated FPLC system ÄKTA Go FPLC Cytiva equipped with a size exclusion column HiPrep Sephacryl S-500 HR column was used to purify lipoprotein-cholesterol nanoparticles. The samples were dispersed in Dulbecco's phosphate-buffered saline PBS 1X pH 7.4 and were eluted in PBS 1X at a constant flow rate of 0.5 mL/min. 1ml of the sample was injected into the column using a coil loop. The sample absorption at 280 nm was automatically recorded and then used to collect desired fractions. The fraction volume was kept constant at 3 ml throughout the runs.

### Negative staining-Transmission Electron Microscopy

Samples of cholesterol-rich lipoprotein nanoparticles were diluted to 0.8 mg/ml (optical OD at 280 nm 0.5) with Dulbecco's phosphate-buffered saline buffer (PBS) 1x and then stained by aqueous uranyl acetate (0.5 wt%) on a carbon film grid. The dry samples were imaged by transmission electron microscopy (TEM) operated at 200KV. The acquired images were analyzed by imaged v. 1.53.

### Results

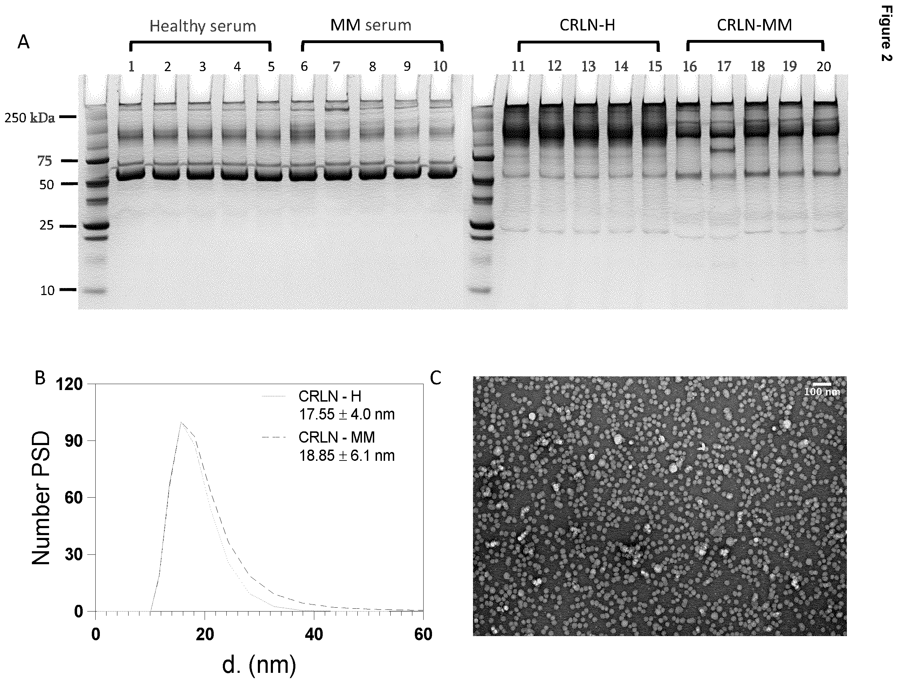

From the SDS-PAGE gel (Figure 2A), serum from five melanoma cancer patients and serum from five pooled healthy donors showed similar protein migration pattern; the most abundant protein is the one between 50 kDa and 75 kDa, which as we suspect, is human serum albumin (HSA) with molecular weight around 67 kDa. While the CRLN extracted both from healthy donors and cancer patients' serum showed different migration patterns compared to serum itself, the most abundant serum protein HSA was depleted dramatically. And except most proteins on the nanoparticles were stuck in the well, there are two major protein bands, one is at around 100 to 150 kDa, and the other one clearly showed at around 25 kDa, while at the same position, all serum samples didn't display this protein band at all. Thus, by extracting CRLN, we can not only deplete the most abundant inert protein HSA but also accumulate small molecular weight proteins, which will potentially increase the chance to detect cancer biomarkers.

Dynamic light scattering characterization of both healthy and cancer CRLN (Figure 2B) showed similar nanoparticle sizes around 17-19 nm. Figure 1C showed the visualization of the CRLN by a negative stained TEM image. From the image, we can see that these CRLN are homogeneous with the size around 20 nm, which is consistent with the measurement of DLS, and the majority of the nanoparticles are monomers.

### Example 2 - The Advantage of CRLN instead of serum

### Materials

Melanoma Cancer patients' serum are from our collaborator Prof. Daniel Speiser (UNIL, Switzerland). In total 5 samples, each sample contains 1 mL serum (two from female age 39 and 44, three from male age 28, 29, and 59, the cancer stage is late stage). Protein BSA and pooled serum from healthy donors were purchased from Sigma Aldrich (Missouri, United States). Chemical polyethylene glycol (PEG) 10000 was purchased from Sigma Aldrich (Missouri, United States). PBS pH 7.4 (1X) was purchased from Life Technology (California, United States). Unless otherwise noted, all chemical and biological reagents were used as received. All solvents purchased were reagent grade.

### Methods

### Extraction of lipoprotein-cholesterol nanoparticles

1 mL serum sample was first to dilute 10 times into 10 mL with PBS 7.4 1X, and then centrifuge under 12000 rpm for 30 mins to remove big aggregates in the serum. After centrifugation, serum was first filtered with 0.22 µm membrane and then adding 1/5 volume of serum (2 mL) 50% (W/V) PEG 10K, mix well, and leave it at 4 degrees for 1 hour. Centrifuge the serum with PEG mixture under 2000 rpm for 15 mins and discard the supernatant. Resuspend the precipitates with PBS 7.4 1X into 5 mL, adding 1/5 volume of the serum solution (1 mL) 50% (W/V) PEG 10K; the second time, mix well and leave it at 4 degrees for 30 mins. Centrifuge the serum with PEG mixture under 2000 rpm for 15 mins, and discard the supernatant. Resuspend the precipitates with PBS 7.4 1X into 1 mL for further FPLC sec column purification.

### Fast protein liquid chromatography purification

An automated FPLC system ÄKTA Go FPLC Cytiva equipped with a size exclusion column HiPrep Sephacryl S-500 HR column was used to purify lipoprotein-cholesterol nanoparticles. The samples were dispersed in Dulbecco's phosphate-buffered saline PBS 1X pH 7.4 and were eluted in PBS 1X at a constant flow rate of 0.5 mL/min. 2ml of the sample was injected into the column using a coil loop. The sample absorption at 280 nm was automatically recorded and then used to collect desired fractions. The fraction volume was kept constant at 3 ml throughout the runs.

### Mass spectrometry

### Sample preparation for Mass Spectrometry

Mass spectrometry-based proteomics-related experiments were performed by the Proteomics Core Facility at EPFL. Each sample was digested by filter aided sample preparation (FASP) [Wiśniewski, J., et al. Nat Methods 6, 359-362 (2009).] with minor modifications. All buffer exchanges were performed on a bench centrifuge at 10'000 rpm. Proteins (Serum, Nanoparticle and HDL samples initial protein content: 20µg; LDL samples initial protein content: 15µg) were reduced with 10 mM TCEP in 8M Urea, 0.1M Tris-HCl pH 8.0 at 37oC for 60min and further alkylated with 40mM Chloroacetamide in 8M Urea, 0.1M Tris-HCl pH 8.0 at 37oC for 45min light protected. Proteins were digested overnight at 37°C using 1/50 w/w enzyme-to-protein ratio with a combination of mass spectrometry grade trypsin (Pierce) and LysC (Wako) supplemented with 10mM CaCl2. Generated peptides were eluted sequentially with 3 x 50µl of 4% TFA and desalted on C18 Empore StageTips using the standard protocol [Rappsilber, J., et al, Y. Nat Protoc 2, 1896-1906 (2007)]. Purified peptides were dried down by vacuum centrifugation. For TMT labeling, dried peptides were first reconstituted in 10µl 100mM HEPES pH 8 and 4µl of TMT solution (25µg/µl in pure acetonitrile) was then added. TMT Labelling was performed at room temperature for 90min, and reactions were quenched with hydroxylamine (0.3% v/v final) for 10min. A minor fraction of TMT-labeled samples was then pooled at a 1:1 ratio across all samples. A single shot control LC-MS run was performed to ensure similar peptide mixing across each TMT channel to avoid the need of further excessive normalization. Quantities of each TMT-labeled sample were adjusted according to the control run. The combined samples were then desalted using a 100mg SEP-PAK C18 cartridge according to provider recommendations and vacuum centrifuged. Pooled samples were fractionated into 12 fractions using an Agilent OFF-Gel 3100 system following the manufacturer's instructions. Resulting fractions were desalted again using SDB-RPS Empore StageTips and dried by vacuum centrifugation.

### LC-MS/MS analysis

Each individual fraction was resuspended in 2% Acetonitrile; 0.1% Formic acid and nano-flow separations were performed on a Dionex Ultimate 3000 RSLC nano UPLC system on-line connected with an Exploris 480 Orbitrap Mass Spectrometer. A capillary precolumn (Acclaim Pepmap C18; 3µm-100Å; 2cm x 75µm ID) was used for sample trapping and cleaning. Analytical separations were performed at 250nl/min over a 150min. biphasic gradients on a 50cm long in-house packed capillary column (75µm ID; ReproSil-Pur C18-AQ 1.9µm silica beads; Dr. Maisch). Acquisitions were performed through Top Speed Data-Dependent acquisition mode using 3 seconds cycle time. First MS scans were acquired at a resolution of 120'000 (at 200m/z) and the most intense parent ions were selected and fragmented by High energy Collision Dissociation (HCD) with a Normalized Collision Energy (NCE) of 36% using an isolation window of 0.7m/z. Fragmented ions scans were acquired with a resolution of 45'000 (at 200m/z) and selected ions were then excluded for the following 45s.

### Data analysis

Raw data were processed using SEQUEST, Mascot, MS Amanda [Dorfer V, et al. J Proteome Res., 13, 3679-84 (2014).] and MSFragger [doi: 10.1038/nmeth.4256.] in Proteome Discoverer v.2.4 against the Uniprot Human Reference Proteome (LM210129, 77'027 entries). Enzyme specificity was set to Trypsin and a minimum of six amino acids was required for peptide identification. Up to two missed cleavages were allowed and a 1% FDR cut-off was applied both at peptide and protein identification levels. For the database search, carbamidomethylation (C), TMT tags (K and Peptide N termini) were set as fixed modifications whereas oxidation (M) was considered as a variable. Resulting text files were processed through in-house written R scripts (version 3.6.3). Two steps of normalization were applied. The first step of normalization was the sample loading normalization [Plubell DL, et al. Mol Cell, Proteomics 16, 873-890 (2017).]. Assuming that total protein abundances were equal across the TMT channels, the reporter ion intensities of all spectra were summed, and each channel was scaled according to this sum, so that the sum of reporter ion signals per channel equals the average of the signals across samples. Then, the Trimmed M-Mean normalization step was also applied using the package EdgeR [Robinson MD, et al., Bioinformatics 26, 139-40 (2010).] (Version 3.26.8). Assuming that the majority of proteins in the samples are non-differentially abundant, this second step calculates normalization factors according to these presumed unchanged protein abundances. During this process, proteins with high or low abundances and proteins with larger or smaller fold-changes were not considered. Differential protein expression analysis was performed using the R bioconductor package limma (version 3.40.6) [Ritchie ME, et al. Nucleic Acids Res., 43, e47 (2015)], followed by the Benjamini-Hochberg multiple-testing correction method [Klipper-Aurbach Y, et al. Med Hypotheses., 45, 486-90 (1995)]. Adjusted P values lower than 0.05 (FDR < 0.05) were considered as significant.

### Results

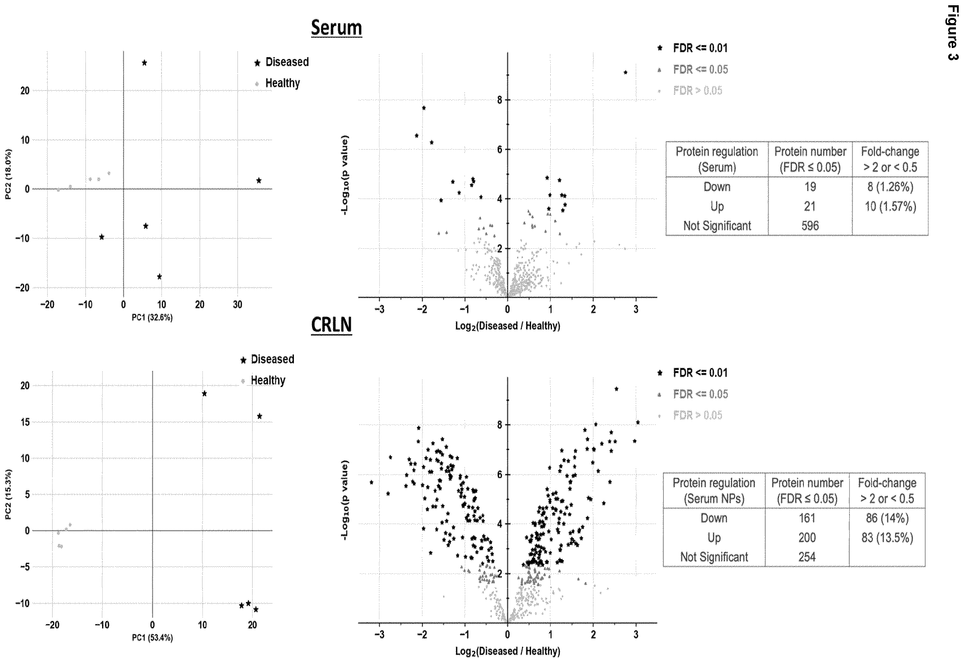

As shown in Figure 3, a principal component analysis (PCA) of the proteomic results of the patients against the pooled serum of healthy people find almost no difference over the two main axes, while analysis applied to the CRLN shows stark differences between the healthy and the diseased group. A volcano plot of all proteins found reveals a very small number of proteins that are either over- or under-expressed in diseased people (21 and 19 respectively), thus even a deeper PCA would probably lead to a poor differentiation between the two groups. All these results are in line with the fact that the most abundant protein albumin played a major effect. A completely different picture is found when comparing CRLN extracted from healthy and diseased people. In such case, a simple PCA clearly distinguished the two categories (Fig. 3), and in a stunning manner of the volcano plot finds 361 proteins that are either over- or under-expressed. One can state that these proteins are the fingerprints of cancer.

### Example 3 - CRLN-Proteomic Analysis in Colon Carcinoma (CC), Metastatic Melanoma (MM) and Prostate Cancer (PC)

### Materials

Colon Carcinoma (CC), Metastatic Melanoma (MM) and Prostate Cancer (PC) serum are from our collaborator Prof. Daniel Speiser (UNIL, Switzerland). Each sample contains 1 mL serum. Chemical polyethylene glycol (PEG) 10000 was purchased from Sigma Aldrich (Missouri, United States). PBS pH 7.4 (1X) was purchased from Life Technology (California, United States). Unless otherwise noted, all chemical and biological reagents were used as received. All solvents purchased were reagent grade.

### Methods

### Extraction of lipoprotein-cholesterol nanoparticles

1 mL serum sample was first to dilute 10 times into 10 mL with PBS 7.4 1X, and then centrifuge under 12000 rpm for 30 mins in order to remove big aggregates in the serum. After centrifugation, serum was first filtered with 0.45 µm low protein binding membrane and then adding 1/5 volume of serum (2 mL) 50% (W/V) PEG 10K, mix well, and leave it at 4 degrees for 1.5 hour. Centrifuge the serum with PEG mixture under 4000 rpm for 15 mins, and discard the supernatant. Resuspend the precipitates with PBS 7.4 1X into 5 mL, adding 1/5 volume of the serum solution (1 mL) 50% (W/V) PEG 10K; the second time, mix well and leave it at 4 degrees for 45 mins. Centrifuge the serum with PEG mixture under 4000 rpm for 15 mins, and discard the supernatant. Resuspend the precipitates with PBS 7.4 1X into 1 mL for further Fast Protein Liquid Chromatography (FPLC, ÄKTA Go) SEC column purification.

### Fast protein liquid chromatography purification

An automated FPLC system ÄKTA Go FPLC Cytiva equipped with a size exclusion column HiPrep Sephacryl S-500 HR column was used to purify lipoprotein-cholesterol nanoparticles. The samples were dispersed in Dulbecco's phosphate-buffered saline PBS 1X pH 7.4 and were eluted in PBS 1X at a constant flow rate of 0.5 mL/min. 1ml of the sample was injected into the column using a coil loop. The sample absorption at 280 nm was automatically recorded and then used to collect desired fractions. The fraction volume was kept constant at 0.8 ml throughout the runs.

### Mass spectrometry

### Sample preparation for Mass Spectrometry

Mass spectrometry-based proteomics-related experiments were performed by the Proteomics Core Facility at EPFL. Each sample was digested by filter aided sample preparation (FASP) [Wiśniewski, J., et al. Nat Methods, **6,** 359-362 (2009).] with minor modifications. All buffer exchanges were performed on a bench centrifuge at 10'000 rpm. Proteins (Serum, Nanoparticle and HDL samples initial protein content: 20µg; LDL samples initial protein content: 15µg) were reduced with 10 mM TCEP in 8M Urea, 0.1M Tris-HCl pH 8.0 at 37oC for 60min and further alkylated with 40mM Chloroacetamide in 8M Urea, 0.1M Tris-HCl pH 8.0 at 37oC for 45min light protected. Proteins were digested overnight at 37°C using 1/50 w/w enzyme-to-protein ratio with a combination of mass spectrometry grade trypsin (Pierce) and LysC (Wako) supplemented with 10mM CaCl2. Generated peptides were eluted sequentially with 3 x 50µl of 4% TFA and desalted on C18 Empore StageTips using the standard protocol [Rappsilber, J., et al, Y. Nat Protoc., 2, 1896-1906 (2007)]. Purified peptides were dried down by vacuum centrifugation. For TMT labeling, dried peptides were first reconstituted in 10µl 100mM HEPES pH 8 and 4µl of TMT solution (25µg/µl in pure acetonitrile) was then added. TMT Labelling was performed at room temperature for 90min, and reactions were quenched with hydroxylamine (0.3% v/v final) for 10min. A minor fraction of TMT-labeled samples was then pooled at a 1:1 ratio across all samples. A single shot control LC-MS run was performed to ensure similar peptide mixing across each TMT channel to avoid the need of further excessive normalization. Quantities of each TMT-labeled sample were adjusted according to the control run. The combined samples were then desalted using a 100mg SEP-PAK C18 cartridge according to provider recommendations and vacuum centrifuged. Pooled samples were fractionated into 12 fractions using an Agilent OFF-Gel 3100 system following the manufacturer's instructions. Resulting fractions were desalted again using SDB-RPS Empore StageTips and dried by vacuum centrifugation.

### LC-MS/MS analysis

Each individual fraction was resuspended in 2% Acetonitrile; 0.1% Formic acid and nano-flow separations were performed on a Dionex Ultimate 3000 RSLC nano UPLC system on-line connected with an Exploris 480 Orbitrap Mass Spectrometer. A capillary precolumn (Acclaim Pepmap C18; 3µm-100Å ; 2cm x 75µm ID) was used for sample trapping and cleaning. Analytical separations were performed at 250nl/min over a 150min. biphasic gradients on a 50cm long in-house packed capillary column (75µm ID; ReproSil-Pur C18-AQ 1.9µm silica beads; Dr. Maisch). Acquisitions were performed through Top Speed Data-Dependent acquisition mode using 3 seconds cycle time. First MS scans were acquired at a resolution of 120'000 (at 200m/z) and the most intense parent ions were selected and fragmented by High energy Collision Dissociation (HCD) with a Normalized Collision Energy (NCE) of 36% using an isolation window of 0.7m/z. Fragmented ions scans were acquired with a resolution of 45'000 (at 200m/z) and selected ions were then excluded for the following 45s.

### Data analysis

Raw data were processed using SEQUEST, Mascot, MS Amanda [Dorfer V, et al. J Proteome Res., 13, 3679-84 (2014).] and MSFragger [doi: 10.1038/nmeth.4256.] in Proteome Discoverer v.2.4 against the Uniprot Human Reference Proteome (LM210129, 77'027 entries). Enzyme specificity was set to Trypsin and a minimum of six amino acids was required for peptide identification. Up to two missed cleavages were allowed and a 1% FDR cut-off was applied both at peptide and protein identification levels. For the database search, carbamidomethylation (C), TMT tags (K and Peptide N termini) were set as fixed modifications whereas oxidation (M) was considered as a variable. Resulting text files were processed through in-house written R scripts (version 3.6.3). Two steps of normalization were applied. The first step of normalization was the sample loading normalization [Plubell DL, et al. Mol Cell Proteomics, 16, 873-890 (2017).]. Assuming that total protein abundances were equal across the TMT channels, the reporter ion intensities of all spectra were summed, and each channel was scaled according to this sum, so that the sum of reporter ion signals per channel equals the average of the signals across samples. Then, the Trimmed M-Mean normalization step was also applied using the package EdgeR [Robinson MD, et al. Bioinformatics, 26, 139-40 (2010).] (Version 3.26.8). Assuming that the majority of proteins in the samples are non-differentially abundant, this second step calculates normalization factors according to these presumed unchanged protein abundances. During this process, proteins with high or low abundances and proteins with larger or smaller fold-changes were not considered. Differential protein expression analysis was performed using the R bioconductor package limma (version 3.40.6) [Ritchie ME, et al. Nucleic Acids Res,. 43, e47 (2015)], followed by the Benjamini-Hochberg multiple-testing correction method [Klipper-Aurbach Y, et al. Med Hypotheses., 45, 486-90 (1995)]. Adjusted P values lower than 0.05 (FDR < 0.05) were considered as significant.

### Results

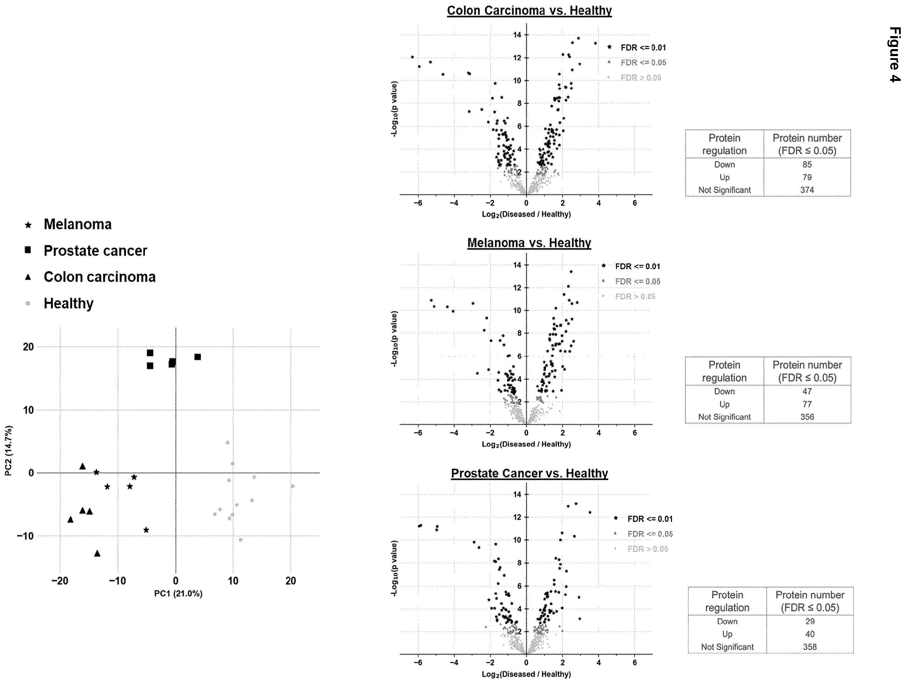

As shown in Figures 4, a three-component PCA displays a clear separation between healthy individuals (light grey dot) and different cancer patients: Melanoma (black star), Prostate cancer (black cubic), and Colon carcinoma (black triangle). The volcano plot of all proteins found, reveals a good manner to find 69 to 164 proteins that are differentially expressed for cancer patients compared to healthy donors. Among them, while comparing Colon carcinoma patients to healthy donors, there are 85 proteins down-regulated and 79 proteins up-regulated; while comparing Melanoma to healthy donors, there are 47 proteins down-regulated and 77 proteins up-regulated; and while comparing Prostate cancer to healthy donors, it shows 29 proteins down-regulated and 40 proteins up-regulated. These proteins are in charge of biological process, immune response or signaling pathways.

### Example 4 - Size Distribution / Fractogram spectrum of CRLN from Healthy and different Cancers Materials

Colon Carcinoma (CC), Metastatic Melanoma (MM) and Prostate Cancer (PC) serum are from our collaborator Prof. Daniel Speiser (UNIL, Switzerland). Healthy samples from the blood bank (Lausanne, Switzerland). Each sample contains 1 mL serum. Chemical polyethylene glycol (PEG) 10000 was purchased from Sigma Aldrich (Missouri, United States). PBS pH 7.4 (1X) was purchased from Life Technology (California, United States). Unless otherwise noted, all chemical and bio-logical reagents were used as received. All solvents purchased were reagent grade.

### Methods

### Extraction of lipoprotein-cholesterol nanoparticles

1 mL serum sample was first to dilute 10 times into 10 mL with PBS 7.4 1X, and then centrifuge under 12000 rpm for 30 mins to remove big aggregates in the serum. After centrifugation, serum was first filtered with 0.45 µm low protein binding membrane and then adding 1/5 volume of serum (2 mL) 50% (W/V) PEG 10K, mix well, and leave it at 4 degrees for 1.5 hour. Centrifuge the serum with PEG mixture under 4000 rpm for 15 mins, and discard the supernatant. Resuspend the precipitates with PBS 7.4 1X into 5 mL, adding 1/5 volume of the serum solution (1 mL) 50% (W/V) PEG 10K; the second time, mix well and leave it at 4 degrees for 45 mins. Centrifuge the serum with PEG mixture under 4000 rpm for 15 mins, and discard the supernatant. Resuspend the precipitates with PBS 7.4 1X into 1 mL for further Fast Protein Liquid Chromatography (FPLC, ÄKTA Go) SEC column purification.

### Fast protein liquid chromatography purification

An automated FPLC system ÄKTA Go FPLC Cytiva equipped with a size exclusion column HiPrep Sephacryl S-500 HR column was used to purify lipoprotein-cholesterol nanoparticles. The samples were dispersed in Dulbecco's phosphate-buffered saline PBS 1X pH 7.4 and were eluted in PBS 1X at a constant flow rate of 0.5 mL/min. 1ml of the sample was injected into the column using a coil loop. The sample absorption at 280 nm was automatically recorded and then used to collect desired fractions. The fraction volume was kept constant at 0.8 ml throughout the runs.

### Results

A fractogram from an FPLC experiment represents the absorbance of different components as a function of their elution time. Each peak in the fractogram corresponds to a different proteins or protein groups eluting from the chromatographic column at a specific retention time. The shape, height, and position of each peak in the fractogram provide the distribution of the proteins in the sample. In the current technique, the proteins are detected by absorbance at a wavelength of 280nm. As the serum samples were run with the same conditions across the whole studies, the number of peaks, peak shape, peak width, and retention time are parameters that can be used to distinctly differentiate the difference among samples and identify them.

The general observation for healthy samples is that they contain two main peaks (at 85 mL, and at 97 mL respectively), and these indicate the presence of two primary groups of different proteins or biomolecules in the samples. Each peak represents a distinct component that has interacted differently with the stationary phase and elutes at a specific time. The first peak represent larger species, which are the CRLN. The interpretation is confirmed by negative staining transmission electron microscopy. As each specific retention time is influenced by the molecular size, charge, hydrophobicity, and interactions of the components with the stationary phase, the later eluting peak is interpreted as being associated with much smaller proteins or protein segments. The conclusion was supported similarly by negative staining transmission electron microscopy. The shape of each peak depends on the interaction of each component with the column. The healthy serum samples have skewed Gaussian profiles. The primary peak is higher which represents the higher concentration or higher abundance of the eluting CRLN than smaller proteins and segments from the second peak.

The finding for the MM samples analyzed is that their fractograms contain four distinct peaks (at 43mL, 78 mL, 97 mL, a 125 mL), and they indicate the presence of four groups of different proteins or biomolecules in the samples. The first peak represents largest species, which are assumed to be exosomes and submicron particles. The second and the third peaks belong to CRLN and small proteins as mentioned above for healthy samples, while the last peak represents the sub-protein species or very small peptides. While the height of the first peak varies widely from sample to sample, its width remains essentially unchanged. In this set of samples, the height and width of the two middle peaks are similar, a feature that distinctly different from those of healthy samples. In addition, the fourth peak is not substantial. Together, all the features of the MM samples in combination show appreciably distinct departure from heathy samples.

The discovery for PC samples is that their fractograms contain two broad peaks (at 85mL, and 97mL, respectively), and they indicate the presence of two groups of variety of proteins or biomolecules in the samples. The first peak represents the principal component of the first group, which are separated as CRLN, and the second peak comprises small proteins and subprotein species. A distinct feature of this set of sample is that the height of the two peaks are closely equivalent. These features of the PC samples set them apart from heathy samples and other groups of samples in the studies.

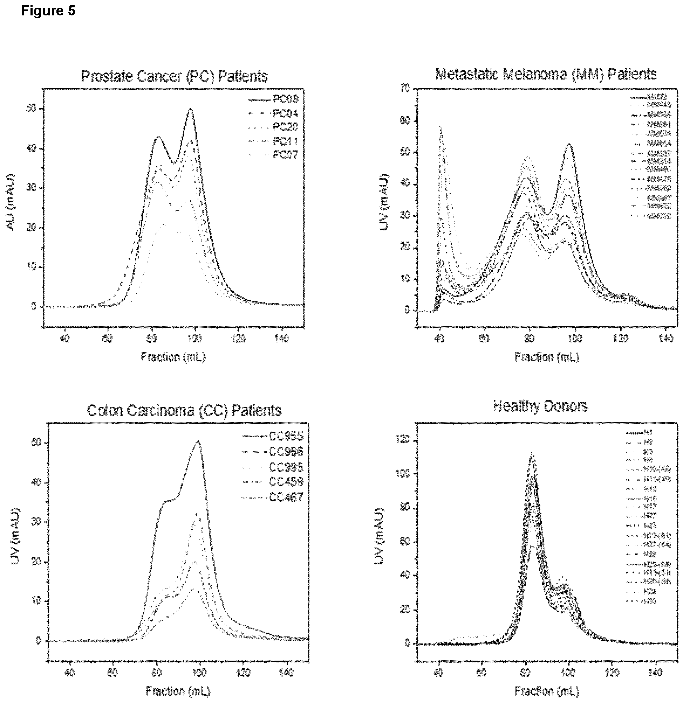

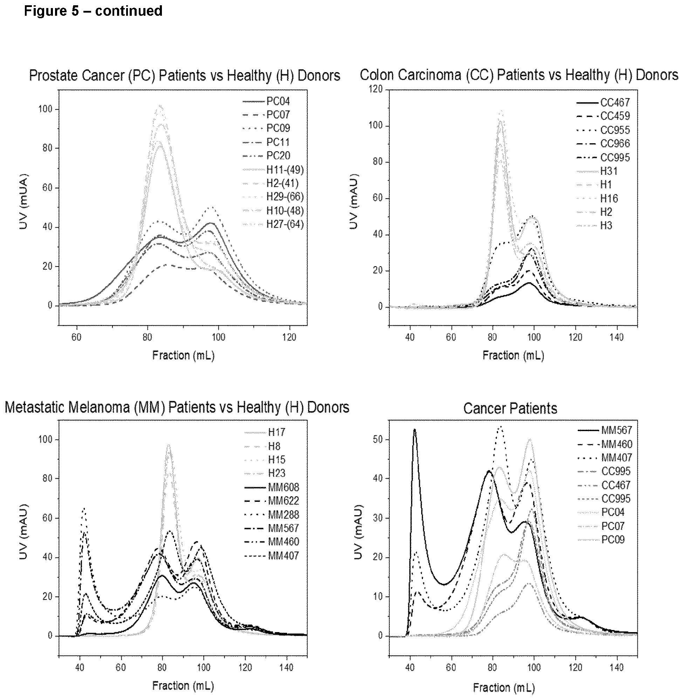

The fractograms of the CC samples is substantially different from all other samples, in that they contain two consecutive broad peaks (at 85mL, and 97 mL, respectively), but the first peak is lower and has longer front trail than the second peak. The first peak is composed of CRLN particles but with broad size distribution, and the second peak is composed of small proteins and subprotein species. The fractograms of CC samples departs from all healthy samples and other groups of cancer samples. Side by side comparison of each group of cancer samples versus healthy samples are displayed in Figure 5. Bases on the above description of retention time, peak shape, peak height, and peak width, each groups of cancer samples are strongly distinguishable from healthy samples. Similarly, when the fractograms of all groups are compared, we find that each group (one healthy, and the three types of cancers tested) can be distinguished from each other.

### Example 5

### Materials

Melanoma Cancer patients' serum were obtained from our collaborator Prof. Daniel Speiser (UNIL, Switzerland). In total 5 samples, each sample contains 1 mL serum (two from female age 39 and 44, three from male age 28, 29, and 59, the cancer stage is late stage). Chemical polyethylene glycol (PEG) 10000 was purchased from Sigma Aldrich (Missouri, United States). PBS pH 7.4 (1X) was purchased from Life Technology (California, United States). Unless otherwise noted, all chemical and biological reagents were used as received. All solvents purchased were reagent grade. A commercial LDL/VLDL and HDL purification kit to extract high density lipoproteins (HDL) and low density lipoprotein (LDL) was purchased from Cell Biolabs Inc. The extraction of HDL and LDL was done following the protocol accompanying the kit.

### Methods

### Extraction of HDL and LDL particles

The extraction of HDL and LDL was done following the protocol accompanying the kit as follows.

Preparation of Reagents: 1X Precipitation Solution B: Dilute the 10X Precipitation Solution B to 1X with deionized water. Stir to homogeneity. Store unused solution at 4°C. 1X Precipitation Solution C: Dilute the 10X Precipitation Solution C to 1X with deionized water. Stir to homogeneity. Store unused solution at 4°C. HDL Resuspension Buffer: Dilute Dextran Solution 1:100 and Precipitation Solution A 1:10 in Tris Solution. For example, add 50 µL of Dextran Solution and 0.5 mL of Precipitation Solution A to 4.45 mL of Tris Solution. Stir to homogeneity. 1X HDL Wash Solution: Dilute the 5X HDL Wash Solution to 1X with deionized water. Stir to homogeneity. To 10 mL of serum or plasma on ice, add 50 µL of Dextran Solution and 500 µL of precipitation solution A. Incubate 5 minutes on ice, followed by spinning at 6000 x g 10 minutes at 4°C. Remove the supernatant, which contains HDL. The remaining pellet which contains LDL.

### LDL Purification

1. Resuspend the pellet above with 400 µL of Bicarbonate Solution and spin a 6000 x g, 10 minutes at 4°C. 2. Transfer the supernatant to a new tube. Discard the pellet. 3. Add 10 mL of 1X Precipitation Solution B to the supernatant. Mix thoroughly by pipetting up and down. 4. Spin at 6000 x g for 10 minutes at 4°C. 5. Discard the supernatant and resuspend the pellet with 200 µL of NaCl Solution. 6. Add 10 mL of 1X Precipitation Solution C. Mix thoroughly by pipetting up and down. 7. Spin at 6000 x g for 10 minutes at 4°C. 8. Repeat steps 5-7. 9. Resuspend the pellet in 200 µL of NaCl Solution (final volume about 500 µL). 10. Add 80 µL of Dextran Removal Solution. Mix thoroughly by pipetting up and down and incubate 1 hour at 4°C. 11. Spin at 6000 x g for 10 minutes at 4°C. 12. Recover the supernatant (purified LDL) and transfer to a new tube. 13. Dialyze the purified LDL in PBS and determine the protein concentration.

### HDL Purification

1. To 10 mL of supernatant from section I above, add 600 µL of Dextran Solution and 1.5 mL of Precipitation Solution A. Incubate for 2 hours at room temperature. 2. Spin 18,000-20,000 x g for 30 minutes at 4°C. 3. Discard supernatant and resuspend pellet in 5 mL of HDL Resuspension Buffer (see Preparation of Reagents Section). Mix thoroughly by pipetting up and down. 4. Spin 6000 x g for 10 minutes at 4°C. 5. Discard supernatant and resuspend pellet in 6 mL of 1X HDL Wash Solution (see Preparation of Reagents Section). 6. Shake for 30 minutes at 4°C. Shaking speed should be sufficient to dissolve pellet, but not sovigorous that bubbles form. 7. Spin 6000 x g for 10 minutes at 4°C. 8. Transfer the supernatant to a new tube and add 900 µL of Dextran Removal Solution. Mix thoroughly by pipetting up and down. 9. Incubate for 1 hour at 4°C. 10. Spin 6000 x g for 10 minutes at 4°C. 11. Transfer the supernatant (containing purified HDL) to a new tube. 12. Dialyze the purified HDL in PBS and determine the protein concentration.

### Mass spectrometry

### Sample preparation for Mass Spectrometry

Mass spectrometry-based proteomics-related experiments were performed by the Proteomics Core Facility at EPFL. Each sample was digested by filter aided sample preparation (FASP) [Wiśniewski, J., et al. Nat Methods **6,** 359-362 (2009).] with minor modifications. All buffer exchanges were performed on a bench centrifuge at 10'000 rpm. Proteins (Serum, Nanoparticle and HDL samples initial protein content: 20µg; LDL samples initial protein content: 15µg) were reduced with 10 mM TCEP in 8M Urea, 0.1M Tris-HCl pH 8.0 at 37oC for 60min and further alkylated with 40mM Chloroacetamide in 8M Urea, 0.1M Tris-HCl pH 8.0 at 37oC for 45min light protected. Proteins were digested overnight at 37°C using 1/50 w/w enzyme-to-protein ratio with a combination of mass spectrometry grade trypsin (Pierce) and LysC (Wako) supplemented with 10mM CaCl2. Generated peptides were eluted sequentially with 3 x 50µl of 4% TFA and desalted on C18 Empore StageTips using the standard protocol [Rappsilber, J., et al, Y. Nat Protoc 2, 1896-1906 (2007)]. Purified peptides were dried down by vacuum centrifugation. For TMT labeling, dried peptides were first reconstituted in 10µl 100mM HEPES pH 8 and 4µl of TMT solution (25µg/µl in pure acetonitrile) was then added. TMT Labelling was performed at room temperature for 90min, and reactions were quenched with hydroxylamine (0.3% v/v final) for 10min. A minor fraction of TMT-labeled samples was then pooled at a 1:1 ratio across all samples. A single shot control LC-MS run was performed to ensure similar peptide mixing across each TMT channel to avoid the need of further excessive normalization. Quantities of each TMT-labeled sample were adjusted according to the control run. The combined samples were then desalted using a 100mg SEP-PAK C18 cartridge according to provider recommendations and vacuum centrifuged. Pooled samples were fractionated into 12 fractions using an Agilent OFF-Gel 3100 system following the manufacturer's instructions. Resulting fractions were desalted again using SDB-RPS Empore StageTips and dried by vacuum centrifugation.

### LC-MS/MS analysis

Each individual fraction was resuspended in 2% Acetonitrile; 0.1% Formic acid and nano-flow separations were performed on a Dionex Ultimate 3000 RSLC nano UPLC system on-line connected with an Exploris 480 Orbitrap Mass Spectrometer. A capillary precolumn (Acclaim Pepmap C18; 3µm-100Å ; 2cm x 75µm ID) was used for sample trapping and cleaning. Analytical separations were performed at 250nl/min over a 150min. biphasic gradients on a 50cm long in-house packed capillary column (75µm ID; ReproSil-Pur C18-AQ 1.9µm silica beads; Dr. Maisch). Acquisitions were performed through Top Speed Data-Dependent acquisition mode using 3 seconds cycle time. First MS scans were acquired at a resolution of 120'000 (at 200m/z) and the most intense parent ions were selected and fragmented by High energy Collision Dissociation (HCD) with a Normalized Collision Energy (NCE) of 36% using an isolation window of 0.7m/z. Fragmented ions scans were acquired with a resolution of 45'000 (at 200m/z) and selected ions were then excluded for the following 45s.

### Data analysis

Raw data were processed using SEQUEST, Mascot, MS Amanda [Dorfer V, et al. J Proteome Res. 13, 3679-84 (2014).] and MSFragger [doi: 10.1038/nmeth.4256.] in Proteome Discoverer v.2.4 against the Uniprot Human Reference Proteome (LM210129, 77'027 entries). Enzyme specificity was set to Trypsin and a minimum of six amino acids was required for peptide identification. Up to two missed cleavages were allowed and a 1% FDR cut-off was applied both at peptide and protein identification levels. For the database search, carbamidomethylation (C), TMT tags (K and Peptide N termini) were set as fixed modifications whereas oxidation (M) was considered as a variable. Resulting text files were processed through in-house written R scripts (version 3.6.3). Two steps of normalization were applied. The first step of normalization was the sample loading normalization [Plubell DL, et al. Mol Cell Proteomics 16, 873-890 (2017).]. Assuming that total protein abundances were equal across the TMT channels, the reporter ion intensities of all spectra were summed, and each channel was scaled according to this sum, so that the sum of reporter ion signals per channel equals the average of the signals across samples. Then, the Trimmed M-Mean normalization step was also applied using the package EdgeR [Robinson MD, et al. Bioinformatics 26, 139-40 (2010).] (Version 3.26.8). Assuming that the majority of proteins in the samples are non-differentially abundant, this second step calculates normalization factors according to these presumed unchanged protein abundances. During this process, proteins with high or low abundances and proteins with larger or smaller fold-changes were not considered. Differential protein expression analysis was performed using the R bioconductor package limma (version 3.40.6) [Ritchie ME, et al. Nucleic Acids Res. 43, e47 (2015)], followed by the Benjamini-Hochberg multiple-testing correction method [Klipper-Aurbach Y, et al. Med Hypotheses., 45, 486-90 (1995)]. Adjusted P values lower than 0.05 (FDR < 0.05) were considered as significant.

### Results

To demonstrate the efficiency our proposed PEG10K based extraction method, we compared it with a commercial kit to extract HDL and LDL and evaluate the results by proteomic analysis. The same source of healthy donors and 5 melanoma cancer patients' serum were used for extraction of HDL and LDL by this commercial kit method, and proteomic analysis was also performed in the same setting as lipoprotein-cholesterol nanoparticles extracted with our lab-developed extraction method. From the result in figure 6, there are in total 710 quantified proteins in HDL and 701 proteins quantified in LDL. Among HDL quantified proteins, only 21 proteins were upregulated, and 11 proteins were downregulated while comparing cancer diseased vs. healthy ones with FDR ≤ 0.05, and with fold-change > 2 or < 0.5, there are 21 proteins (3% of total quantified proteins in HDL) upregulated and 10 proteins (1.4% of total quantified proteins in HDL) downregulated. Among LDL quantified proteins, there are 48 proteins upregulated and 93 proteins downregulated while comparing cancer diseased vs. healthy ones with FDR ≤ 0.05, and with fold-change > 2 or < 0.5, there are only 44 (6.3 % of total quantified proteins in LDL) upregulated and 81 proteins (11.5% of total quantified proteins in LDL) downregulated. On the extraction yield wise, with a low quantity of serum (1 mL) and following the instruction of the extraction kit, the yield of LDL is very low; on average less than 20 ug LDL/mL serum can be obtained, and different individual serum obtained LDL is very heterogeneous. While using the novel lab-developed extraction method as described herein (also called "PEG10K based extraction method"), from 1 mL each cancer individual serum, we could achieve on average 700ug CRLN/mL serum, around 35 times higher than the commercial kit extraction. And from the proteomic analysis, the number of differentiated proteins from CRLN samples is 361/615, while in LDL samples, the differentiated proteins are 142/701, which indicates the advantages in sensitivity and better accuracy.

## Claims

1. A method for the diagnosis of cancer in a sample of cholesterol-rich lipoprotein nanoparticles obtained from a test subject comprising
(α) determining the size distribution or the fractogram in chromatography of the cholesterol-rich lipoprotein nanoparticles in the sample of cholesterol-rich lipoprotein nanoparticles obtained from the test subject, and
(β) comparing the size distribution or the fractogram of (a) to the corresponding size distribution or fractogram in one or more samples of cholesterol-rich lipoprotein nanoparticles obtained from one or more healthy subjects or a predetermined standard size distribution or fractogram obtained from a corresponding size distribution or fractogram in one or more samples of cholesterol-rich lipoprotein nanoparticles obtained from one or more healthy subjects,
wherein a different size distribution or fractogram in the sample of the test subject as compared to the size distribution or the fractogram in the one or more samples of cholesterol-rich lipoprotein nanoparticles obtained from one or more healthy subjects or the predetermined standard size distribution or fractogram indicates that the test subject has cancer.

2. The method of claim 1, wherein the size distribution of the cholesterol-rich lipoprotein nanoparticles is determined
based on the retention time of the cholesterol-rich lipoprotein nanoparticles in a liquid chromatography, preferably in a fast protein liquid chromatography (FPLC);
by size exclusion chromatography (SEC);
by sodium dodecyl sulfate-polyacrylamide gel electrophoresis (SDS-PAGE);
by differential centrifugal sedimentation;
by analytical ultracentrifugation; or
based on multi-angle dynamic light scattering.

3. The method of claim 1 or 2, further comprising
(γ) comparing the size distribution of (a) to one or more corresponding size distributions from one or more samples of cholesterol-rich lipoprotein nanoparticles, wherein each of the one or more samples has been obtained from one or more cancer subjects having the same type of cancer or one or more predetermined standard size distributions, wherein each predetermined standard size distribution has been obtained from a sample of cholesterol-rich lipoprotein nanoparticles obtained from one or more cancer subjects having the same type of cancer,
wherein a substantially same size distribution in the sample of the test subject as compared to the size distribution of a sample of cholesterol-rich lipoprotein nanoparticles that has been obtained from one or more cancer subjects having the same type of cancer or one of the predetermined standard size distributions indicates that the test subject has the same type of cancer.

4. The method of any one of claims 1 to 3, further comprising prior to step (a) the step (a)' extracting the cholesterol-rich lipoprotein nanoparticles from a serum or plasma sample of the test subject.

5. The method of claim 4, wherein extracting the cholesterol-rich lipoprotein nanoparticles from the serum or plasma sample in step (a)' comprises
(i) diluting the serum or plasma sample at a ratio of between 1:2 to 1:25, preferably 1:5 to 1:15 and most preferably about 1:10 with a physiological buffer,
(ii) passing the diluted serum or plasma through a filter having a pore size of between 50 nm and 2.5 µm, preferably between 100 nm and 1.0 µm, and most preferably between 200 nm and 5000 nm,
(iii) adding 1/2 to 1/10 volume, preferably 1/4 to 1/6 volume and most preferably about 1/5 volume of polyethylene glycol (PEG, preferably having an average molecular weight of 10,000 g/mol and/or at concentration of 50 wt%) to the filtrated diluted serum or plasma in order to obtain a PEG mixture,
(iv) incubating the PEG mixture for at least 30 min, preferably 30 min to 24h, and most preferably 1h to 3h, at a temperature between 0°C and 37°C, preferably 2°C and 25°C and most preferably at about 4°C,
(v) subjecting the incubated PEG mixture to centrifugation under 200 g to 4500 g for at least 5 min, preferably 400 g to 1200 g for 10 min to 20 min and most preferably about 750 g for about 15 min; and
(vi) discarding the supernatant and resuspending the pellet comprising the cholesterol-rich lipoprotein nanoparticles from the serum or plasma sample in a physiological buffer.

6. The method of claim 5, further comprising after step (i) and before step (ii)
(i)' subjecting the diluted serum or plasma to centrifugation under 9500 g to 24000 g for at least 15 min, preferably 8500 g to 24000 g for 15 min to 45 min and most preferably about 16000 g for about 30 min and collecting the supernatant as diluted serum or plasma.

7. The method of claim 5 or 6, further comprising
(vii) adding 1/2 to 1/10 volume, preferably 1/4 to 1/6 volume and most preferably about 1/5 volume of PEG (preferably having an average molecular weight of 10,000 g/mol and/or at concentration of 50 wt%) to the resuspended pellet in order to obtain a second PEG mixture,
(viii) incubating the second PEG mixture for at least 15 min, preferably 15 min to 24h, and most preferably 20 min to 1h, at a temperature between 0°C and 37°C, preferably 2°C and 25°C and most preferably at about 4°C,
(ix) subjecting the incubated PEG mixture to centrifugation under 200 g to 4500 g for at least 5 min, preferably 400 g to 1200 g for 10 min to 20 min and most preferably about 750 g for about 15 min; and
(x) discarding the supernatant and resuspending the second pellet comprising the cholesterol-rich lipoprotein nanoparticles from the serum or plasma sample in a physiological buffer.

8. The method of any one of claims 5 to 7, further comprising subjected the resuspended pellet or the second resuspended pellet to protein liquid chromatography purification for further purification of the sample of cholesterol-rich lipoprotein nanoparticles.

9. The method of any one of claims 5 to 8, wherein the PEG has a molecular weight of 2K to 20K, preferably 5K to 15K and most preferably of about 10K and/or wherein the PEG has a concentration of 25 wt% to 75 wt%, preferably 40 wt% to 60 wt% and most preferably about 50 wt%.

10. The method of any one of claims 5 to 9, wherein the physiological buffer is a bicarbonate or phosphate buffer, preferably phosphate buffered saline (PBS) and most preferably 1x PBS, pH 7.4.

11. The method of any one of claims 2 to 10, wherein the serum or plasma sample has a volume of 50µl to 5mL, preferably 100µl to 2mL, and most preferably 400µl to 1mL.

12. The method of any one of claims 1 to 11, further comprising
(a) determining the protein level of at least one protein in the sample of isolated cholesterol-rich lipoprotein nanoparticles; and
(b) comparing the determined protein level to the corresponding protein level in one or more samples of cholesterol-rich lipoprotein nanoparticles obtained from one or more healthy subjects or a predetermined standard obtained from a corresponding protein level in one or more samples of cholesterol-rich lipoprotein nanoparticles obtained from one or more healthy subjects,
wherein an at least 1.5-fold increase or decrease of the determined protein level as determined in (a) as compared to the protein level in one or more samples of cholesterol-rich lipoprotein nanoparticles obtained from one or more healthy subjects or the predetermined standard indicates that the test subject has cancer, and
wherein steps (a) and (b) are preferably carried out after step (a) and, if step (a)' is present, after step (a)'.

13. The method of any one of claim 12, wherein in step (a) the protein level of at least one protein in the isolated cholesterol-rich lipoprotein nanoparticles is determined by a mass spectrometry-based process.

14. The method of claim 13, wherein the mass spectrometry-based process comprises:
(A) digesting the proteins in the sample of cholesterol-rich lipoprotein nanoparticles into peptides, preferably by one or more proteases and most preferably by trypsin and/or the endoproteinase LysC or LysN;
(B) optionally labeling the peptides by tandem mass tags (TMT);
(C) optionally separating the peptides into fractions; and
(D) analyzing the peptides by mass spectrometry, preferably by LC-MS/MS, thereby determining the protein level of the at least one protein in the sample of isolated cholesterol-rich lipoprotein nanoparticles.

15. The method of any one of claims 12 to 14, wherein the at least one protein is at least 5, preferably at least 10, and most preferably at least 25 proteins.
